# EUROPEAN PATENT SPECIFICATION

(11) **EP 4 180 527 B1**
(45) Date of publication and mention of the grant of the patent: **09.07.2025**
(21) Application number: 22206444.6
(22) Date of filing: 09.11.2022
(51) Int. Cl.: C12N 15/11, C12N 15/113

(54) **SYNTHESIS METHOD OF TARGETED DRUG NCOVSHRNA·2ACE2**
SYNTHESEVERFAHREN FÜR GEZIELTEN WIRKSTOFF NCOVSHRNA·2ACE2
PROCÉDÉ DE SYNTHÈSE DE MÉDICAMENT CIBLÉ NCOVSHRNA·2ACE2

(30) Priority: 11.11.2021 CN 202111329892; 08.02.2022 CN 202210116885; 01.05.2022 CN 202210473225; 09.06.2022 CN 202210643026; 01.08.2022 CN 202210917146
(43) Date of publication of application: 17.05.2023
(73) Proprietor: HANGZHOU CHICHUANG BIOTECHNOLOGY CO., LTD., Hangzhou 311215 (CN)
(72) Inventor: WENG, Binghuan, Hangzhou, 311215 (CN); LI, Lanjuan, Hangzhou, 311215 (CN); WANG, Hui, Hangzhou, 311215 (CN); HE, Lin, Hangzhou, 311215 (CN); ZHU, Zhiyong, Hangzhou, 311215 (CN); SHENG, Guoping, Hangzhou, 311215 (CN); MA, Duan, Hangzhou, 311215 (CN); YAO, Xufeng, Hangzhou, 311215 (CN); YU, Jialing, Hangzhou, 311215 (CN); LUO, Yuqin, Hangzhou, 311215 (CN); SHI, Yue, Hangzhou, 311215 (CN)
(74) Representative: JD&P Patent Attorneys Joanna Dargiewicz & Partners

(56) References cited:
- WO-A1-2021/204216
- CN-A- 113 528 516
- KHANALI JAVAD ET AL: "Nucleic Acid-Based Treatments Against COVID-19: Potential Efficacy of Aptamers and siRNAs", FRONTIERS IN MICROBIOLOGY, vol. 12, 8 November 2021 (2021-11-08), pages 758948, XP093036343, Retrieved from the Internet <URL:https://www.ncbi.nlm.nih.gov/pmc/articles/PMC8630580/pdf/fmicb-12-758948.pdf> DOI: 10.3389/fmicb.2021.758948

## Description

The present disclosure relates to a synthesis method of a targeted drug nCoVshRNA·2ACE2, and belongs to the field of biopharmaceuticals.

Human angiotensin-converting enzyme II (ACE2) is a type I transmembrane glycoprotein expressed by target cells. The ACE2 consists of 805 amino acids, where amino acids 1 to 740 are located extracellularly and called extracellular ACE2; amino acids 741 to 763 are located in a transmembrane region and called transmembrane ACE2; and amino acids 764 to 805 are located intracellularly and called intracellular ACE2.

The structure of a coronavirus includes a single-stranded ribonucleic acid (ssRNA), a spike protein (S protein), a membrane protein (M protein), an envelope protein (E protein), and a nucleocapsid protein (N protein), where an N-terminus of the S protein includes an N-terminus domain (S1-NTD) and a receptor-binding domain (S1-RBD).

The coronavirus binds to the ACE2 of a target cell through its S 1-RBD, and undergoes membrane fusion and endocytosis, such that the coronavirus enters the target cell through an ACE2 channel. This shows that coronavirus RBD and target cell ACE2 have a ligand-receptor relationship. According to this, an ACE2 polypeptide is designed as a targeted delivery vector, and the ACE2 polypeptide and an RNAi sequence are synthesized into nCoVshRNA-2ACE2 for targeted delivery of shRNA by the ACE2. Due to cell penetrating peptide properties of the ACE2, an siRNA drug in the nCoVshRNA-2ACE2 is subjected to targeted delivery by the ACE2 and passes through the ACE2 channel of the target cell, playing a specific role in targeting virus-infected cells; since ACE2 can combine with the virus RBD, nCoVshRNA-2ACE2 combines with the virus to form a complex, such that the siRNA drug can enter target cells with virus infection, playing a characteristic role in virus-infected cells. In addition, since the ACE2 can bind to the RBD, nCoVshRNA-2ACE2 can block the virus RBD, thereby blocking virus infection of the target cells.

RNA interference (RNAi), as an efficient sequence-specific gene silencing technology, is bringing unimaginable application prospects to the treatment of diseases, and a variety of siRNA drugs have been approved and marketed by the Food and Drug Administration (FDA). However, currently siRNA in the siRNA drugs is generally designed using a single strain, the siRNA drugs are prepared using a single-stranded siRNA (antisense RNA), or non-specific delivery of the siRNA drugs is conducted using non-targeted delivery vectors. Moreover, the siRNA generally designed using a single strain may be off-target and ineffective due to the constantly mutating coronavirus. Therefore, it is necessary to compare mutated strains, so as to preferably select an siRNA that is consensus to each strain and does not change with the virus mutation, thereby preparing a broad-spectrum siRNA drug for the mutated and mutating strains. If a non-targeted delivery vector is used, the siRNA drugs may be delivered to cells that are less susceptible to coronavirus infection, since the cells do not express ACE2. Accordingly, it is necessary to design a targeted delivery vector that can specifically deliver the siRNA to target cells. More importantly, according to an RNAi mechanism reported by Hre A et al., a double-stranded RNA prepared by mixing the sense RNA and antisense RNA has an efficiency of silencing homologous mRNAs over 100 times higher than that of the single-stranded RNA (antisense RNA). This shows that a correct and effective RNAi technology should prepare drugs using the double-stranded RNA (shRNA) containing the sense and antisense siRNAs, rather than the single-stranded siRNA.

Small interfering RNA, or siRNA, is delivered to the cytoplasm to regulate gene expression in a manner that participates in RNAi, thus specifically degrading a complementary target messenger RNA (mRNA). Since siRNA is difficult to pass through the cell membrane and is easily degraded by RNases, traditionally the siRNA is transported and protected non-specifically by methods such as lipid nanoparticle encapsulation. In the present disclosure, the siRNA is synthesized into a shRNA, and an ACE2 polypeptide is ligated to double-strand ends of the shRNA to achieve specific transport and protection of the siRNA.

In the prevention and treatment of COVID-19, if nCoVsiRNA can be stably and specifically delivered to target organs, target tissues, and target cells in sequence with a suitable targeted delivery vector. The nCoVsiRNA can localize to target cells, cross the target cell membrane, and be released into the target cytoplasm, which is broadly effective against a variety of variant strains. As a result, a targeted gene therapy for the COVID-19 can be better developed.

To solve the above problems, in the present disclosure, a targeted drug nCoVshRNA·2ACE2 is designed and synthesized.

Khanali Javad et al assess their paper "Nucleic Acid-Based Treatments Against COVID-19: Potential Efficacy of Aptamers and siRNAs", Frontiers in Microbiology, Vol. 12, 8 November 2021, page 758948, DOI: 10.3389/fmicb.2021.758948 the plausibility of using aptamers. siRNAs, and aptamer-siRNA chimeras against the SARS-CoV-2 based on their previously established effectiveness.

WO 2021/204216 A1 discloses a siRNA inhibiting novel corona virus gene expression and a pharmaceutical composition.

An objective of the present disclosure is to provide a drug nCoVshRNA-2ACE2 that delivers a shRNA by using a targeted delivery vector ACE2, and a synthesis method and use thereof. In the present disclosure, the bivalent ACE2 can bind to a RBD to neutralize a virus and deliver the shRNA in a targeted manner, and make the shRNA delivered by the ACE2 to indirectly bind to the RBD to form a complex of "shRNA-ACE2-RBD-virus", such that the shRNA is targeted to be delivered by the complex, and enters target cells with virus infection, thus playing a broad-spectrum and targeted role in resisting variant strains.

The objective of the present disclosure is achieved by a synthesis method according to claim 1.

A target siRNA against the variant strain is selected, a shRNA is synthesized, and then an ACE2 polypeptide is separately extended and ligated to double-strand ends of the shRNA to synthesize a targeted drug nCoVshRNA-2ACE2 of a COVID-19 virus.

Screening of a target against variant strains: the siRNA is selected from a consensus gene of various pathogenic coronaviruses and variant strains thereof, where the consensus gene includes a conserved gene, an ultra-conserved gene, and/or a conserved microsatellite, such that the siRNA is a common target of each variant strain that does not change with virus mutation, with a broad-spectrum effect in resisting the variant strains.

Synthesis of an anti-variant strain target siRNA: the siRNA is synthesized into two complementary oligonucleotide siRNAs of 21 nt to 25 nt, and a base sequence that acts as a spacer is synthesized.

Synthesis of a shRNA: the two complementary oligonucleotide siRNAs and the base sequence that acts as a spacer are further synthesized into a double-stranded small hairpin shRNA with a loop that is in a middle position and separated by the base sequence.

Preferred siRNA: an interference vector is constructed, and mRNA expression, protein expression, and interference effect of the shRNA are detected; after conducting siRNA design, synthesis, screening, iterative design, and verification, the siRNA with a high silencing efficiency is preferably selected.

Synthesis of preferred siRNAs and shRNAs: siRNAs and shRNAs are synthesized using sequences of the preferred siRNA according to the method above, including chemical modifications to increase stability and avoid off-target.

Synthesis of an ACE2 polypeptide or protein: the synthesized ACE2 includes but is not limited to a full-length ACE2, a transmembrane ACE2 of amino acids 741 to 763, an intracellular ACE2 of amino acids 764 to 805, an extracellular ACE2 of amino acids 1 to 740, and an amino acid sequence codon-optimized ACE2 polypeptide or protein.

Synthesis of nCoVshRNA·2ACE2: the shRNA is ligated by a coupling method using a disulfide bond, a phosphodiester bond, a phosphorodithioate bond, a thioether bond, an oxime bond, an amide bond, and a maleimide-thiol bond with the ACE2 to synthesize a compound; alternatively, ACE2-shRNA-ACE2 is directly synthesized from the amino acid level according to a nucleotide sequence of the shRNA and an amino acid sequence of the ACE2.

Purification of the nCoVshRNA-2ACE2: the purification is conducted by high-performance liquid chromatography, reversed high-performance liquid chromatography, or ion exchange chromatography.

Liposome modification of the nCoVshRNA-2ACE2: a liposome-modified compound is prepared by adsorbing the negatively-charged shRNA to a positively-charged liposome; a PEG-internalized liposome-modified compound is prepared by thiolation of an amino group of the ACE2 to form a maleimide-thiol bond between a thiol group and maleamide of the liposome; a liposome-modified compound is prepared by forming a carbamate bond between the amino group of the ACE2 and the liposome; and a liposome-modified compound is prepared by ligating the ACE2 or an ACE2 fragment to the liposome-modified siRNA.

Verification of the nCoVshRNA·2ACE2: an antiviral effect of the nCoVshRNA-2ACE2 on two or more different variant strains is detected at the cellular level *in vitro,* and it is observed whether it has a broad-spectrum anti-variant strain effect targeting the conserved gene; it is tested that whether the nCoVshRNA·2ACE2 has an effect in targeted delivery of the shRNA in animals and whether the nCoVshRNA-2ACE2 can stimulate the host to produce ACE2-Ab.

**The present disclosure has the following beneficial effects:**
In the present disclosure, an siRNA drug is designed that delivers shRNA by targeting ACE2.

In the present disclosure, a difference between the drug and traditional siRNA drugs is that: from various coronaviruses and their variant strains, a common target siRNA of each strain that does not change with the virus mutation is selected, such that the siRNA has a broad-spectrum anti-variant strain effect.

In the present disclosure, a difference between the drug and the traditional siRNA drugs is that: the traditional siRNA drugs adopt a sense strand siRNA or an antisense strand siRNA, that is, a single-stranded siRNA; in the present disclosure, shRNA is synthesized from the siRNA of sense and antisense strands. According to an RNAi mechanism, double-stranded RNA has a more efficient RNAi effect. Therefore, the present disclosure has a more accurate design that is more in line with the RNAi mechanism.

In the present disclosure, a distinguishing feature of the drug from the traditional siRNA drugs is that: based on the characteristics of coronavirus infection through the binding of RBD to ACE2 expressed by host susceptible cells, the nCoVshRNA-2ACE2 that delivers shRNA by targeting ACE2 is designed, which consists of a short hairpin shRNA region and a double-stranded ACE2 region. ACE2 plays the role of delivering shRNA and binding coronavirus RBD. A "shRNA-ACE2-RBD-virus" complex formed by the ACE2 and virus RBD can make shRNA enter the target cells with virus infection. This method of co-delivering shRNA with the ACE2 and virus can avoid a side effect of non-specific delivery of the shRNA to uninfected cells, such that shRNA can produce an anti-variant strain effect against the virus-infected cells.

Since the siRNA/shRNA is negatively charged and lipid-soluble, with poor permeability and stability; after synthesizing the siRNA/shRNA with the ACE2 into the nCoVshRNA-2ACE2, the permeability, stability, and easy delivery of the siRNA/shRNA are optimized.

Furthermore, the nCoVshRNA-2ACE2 is synthesized with 2 molecules of the ACE2 and 1 molecule of the shRNA, where the bivalent ACE2 can bind to the virus RBD, thereby neutralizing the virus and blocking the virus from infecting target cells.

Furthermore, the nCoVshRNA-2ACE2 includes two ACE2 molecules and one shRNA molecule. Since the shRNA is not only antiviral, but also an oligonucleotide immunologic adjuvant, nCoVshRNA-2ACE2 is more antigenic than the single-molecule ACE2, which can stimulate the host to produce higher titer of ACE2-Ab. The ACE2-Ab competes with the virus for binding to an ACE2 receptor of the target cell, thereby enabling the nCoVshRNA-2ACE2 (ACE2-Ab) to block viral infection.

Furthermore, liposome modification can make the shRNA release slowly *in vivo,* prolong a drug efficacy, and play a role in endocytosis into plasma, and can be used as an immunologic adjuvant to enhance an ACE2 immune effect in the nCoVshRNA-2ACE2.

Furthermore, *in vitro* cell experiments show that the nCoVshRNA·2ACE2 is effective against two different variant strains simultaneously, indicating an anti-variant strain effect by targeting conserved genes; *in vivo* animal experiments show that the nCoVshRNA-2ACE2 has an effect in targeted delivery of the RNAi in animals, and the stimulation-derived ACE2-Ab can inhibit virus infection by blocking ACE2 receptors.

### BRIEF DESCRIPTION OF THE DRAWINGS

FIG. 1 shows a technical circuit diagram for preparing nCoVshRNA-2ACE2 in the present disclosure;
FIG. 2 shows a structural representation of the nCoVshRNA-2ACE2 in the present disclosure;
FIG. 3 shows a schematic diagram of the nCoVshRNA·2ACE2 and use thereof in the present disclosure;
FIG. 4 shows a schematic diagram of ACE2-targeted delivery of a liposome-modified shRNA of the present disclosure; and
FIG. 5 shows a schematic diagram of ACE2-targeted delivery of a liposome-modified siRNA of the present disclosure.

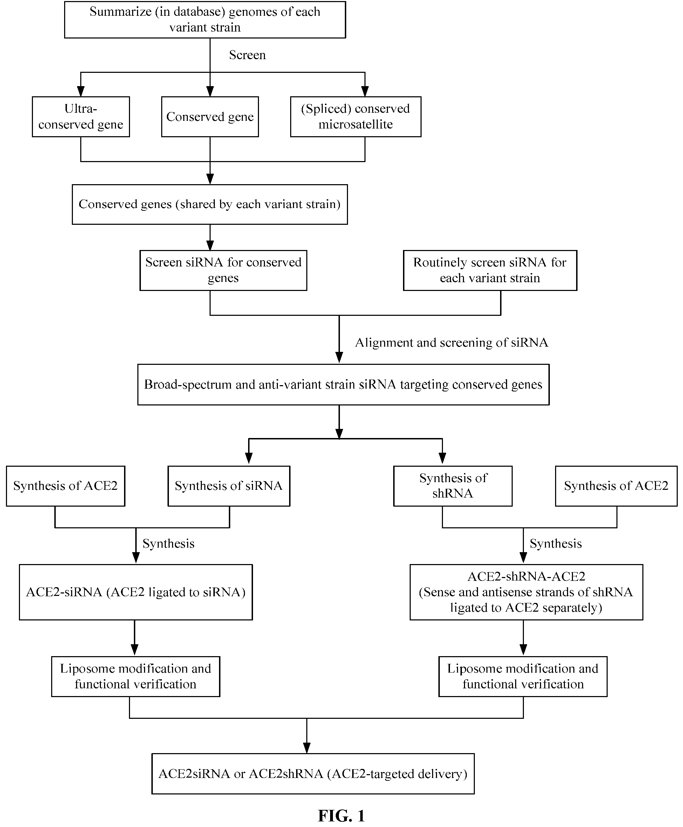

In FIG. 1, after conserved gene screening, broad-spectrum anti-variant strain target siRNA screening by targeting conserved genes, shRNA synthesis, and ACE2 synthesis, the nCoVshRNA·2ACE2 or ACE2shRNA is finally synthesized.

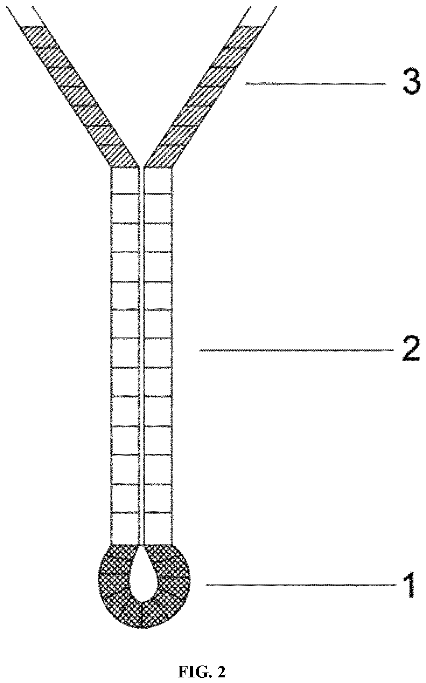

In FIG. 2, 1 is a loop, 2 is a shRNA formed by two complementary sense and antisense strands, and 3 is two ACE2 polypeptides (proteins); the two ACE2 polypeptides are ligated to the sense and antisense strands of the shRNA, respectively. shRNA is protected by ACE2 and then delivered by the ACE2 to the virus RBD or the ACE2 receptor channel, and then specifically enters the target cytoplasm with the virus RBD through the ACE2 channel to degrade the target gene of virus.

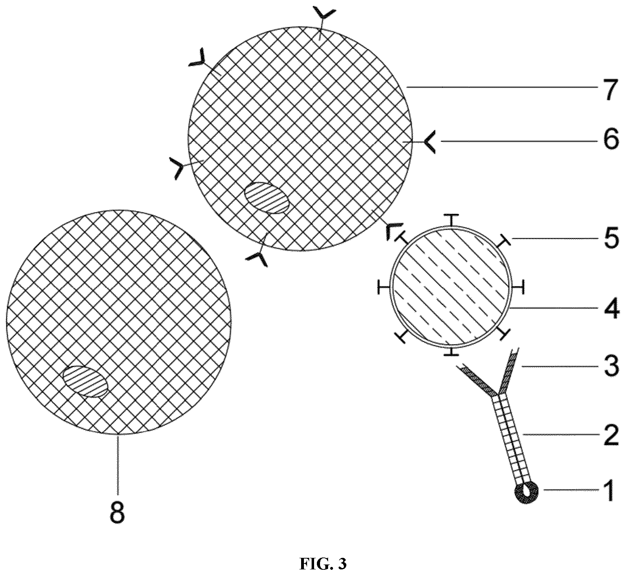

In FIG. 3, 1 is the loop formed by the base sequence between the sense and antisense strands of the small hairpin shRNA; 2 is the shRNA formed by complementary combination of two sense and antisense strands of siRNA, where the siRNA takes the conserved gene of coronavirus as an interference target and has a targeted gene therapy effect of broad-spectrum anti-variant strain properties; 3 is the ACE2 ligated to the sense and antisense strands of the shRNA; 4 is the coronavirus; 5 is the RBD of a coronavirus S protein; 6 is the cells; 7 is the expressed ACE2 receptors; and 8 is the cells that do not express ACE2. As shown in FIG. 2, the nCoVshRNA·2ACE2 is composed of the loop 1, the shRNA 2, and the ACE2 3; the coronavirus 4 infects cells 7 through specific binding of its RBD 5 to ACE2 receptor 6, but the coronavirus 4 does not infect cells 8 that do not express ACE2; ACE2 3 in the nCoVshRNA-2ACE2, like ACE2 6 expressed by cells 7, can also bind to the RBD 5 of coronavirus 4, such that the ACE2 3 can compete with the ACE2 6 for binding to the RBD 5, such that the ACE2 3 has an ability to inhibit the virus 4 from infecting the cells 7; the nCoVshRNA-2ACE2 plays the role of a vaccine in a later stage, since the ACE2 3 can stimulate the host to produce anti-ACE2, and the anti-ACE2 can block the ACE2 6, thereby inhibiting the virus 4 from infecting cells 7; more importantly, through bridging of the ACE2 3, a complex of "shRNA 2-ACE2 3-RBD 5-virus 4" is formed, allowing shRNA 2 to enter cell 7 with virus 4, targeting to interfere with the replication of virus 4 in cells 7, avoiding toxic and side effects caused by the non-specific entry of the shRNA 2 into cells 8 that are not infected with virus 4.

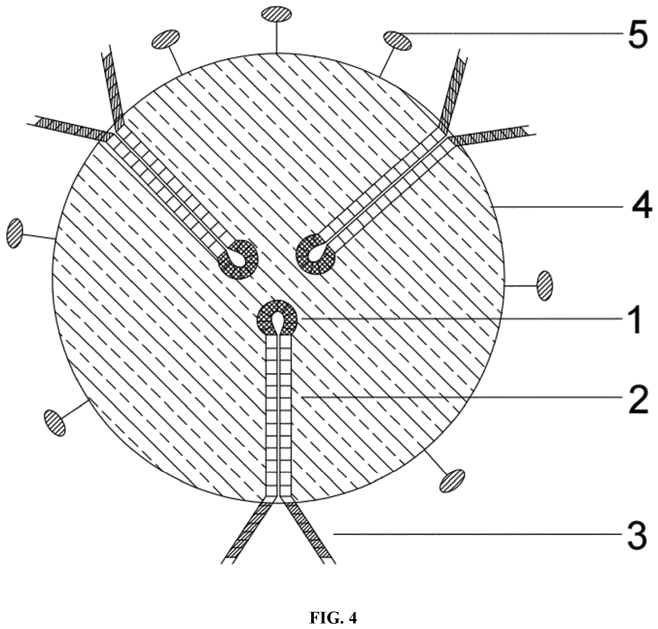

In FIG. 4, 1 and 2 are the loop and shRNA wrapped by liposome 4, respectively, where the shRNA is formed by the complementary combination of two sense and antisense strands of siRNA, and the siRNA takes the coronavirus conserved gene as an interference target; 3 is ACE2 exposed outside the liposome 4, plays a role in targeted delivery; liposome 4 protects shRNA and induces endocytosis; 5 is polyethylene glycol (PEG), the PEG can release siRNA slowly with long-term circulation. As shown in FIG. 3, a complex of ACE2-shRNA/Lip is formed.

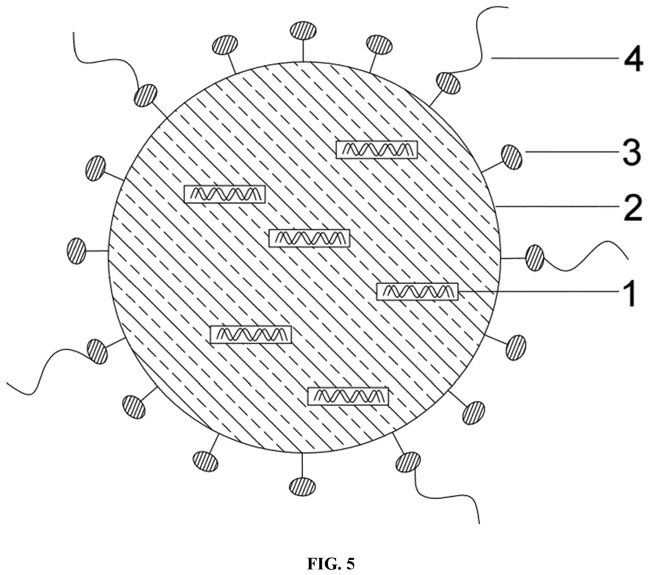

In FIG. 5, 1 is the siRNA encapsulated by liposomes, 2 is a liposome layer, 3 is a PEG layer, and 4 is the ACE2. The siRNA acts as RNAi; the liposome protects siRNA and induces endocytosis; the PEG enables slow release and long-term circulation of siRNA; and the ACE2 is in the outermost layer and plays a role of targeted delivery of the siRNA. As shown in FIG. 4, a complex of ACE2-siRNA/Lip is formed.

### DETAILED DESCRIPTION OF THE EMBODIMENTS

Below in conjunction with accompanying drawings 1, 2, 3, 4 and 5, the specific implementation method of the present disclosure is described in detail, but these exemplary descriptions do not constitute any limitation to the protection scope defined by the claims of the present disclosure.

### I. Design of siRNA by targeting ultra-conserved genes, conserved genes, or conserved microsatellites

### 1. Design of ultra-conserved genes, conserved genes, and conserved microsatellites

As shown in FIG. 1 of the technical circuit, a whole genome sequence (cDNA) of β-coronavirus (especially COVID-19 virus and its variant strains) is downloaded from the Genbank database (http://www.NCBI.nlm.nih.gov/genome/), the longest common subsequence is searched in the whole genome sequence to obtain the ultra-conserved genes or conserved genes; with Clustal W software, sequence alignment is conducted on the whole genome downloaded from the Genbank database, the similarity between different sequences is detected, and conserved microsatellite sequences are screened; with MEGA6.0 molecular evolution genetic analysis software, an amino acid germline molecular evolution tree is constructed by using neighbor-joining (N-J) on the downloaded coronavirus amino acid sequence, and molecular variation characteristics of the amino acid sequence were analyzed and optimized, so as to infer conserved genes sequence.

The following three longest and second longest ultra-conserved subsequences are obtained, with a length of 22 bp to 30 bp, which are comparable to a length of small RNAs, but these three subsequences are not included in higher organisms, especially human beings. A specific sequence is as follows:
SEQ ID NO: 1 (Subsequence 1) = ttaatacgacctctctgttggattttgaca (30 bp);
SEQ ID NO: 2 (Subsequence 2) = ggttcgcaacttcacaca gagt (22 bp); and
SEQ ID NO: 3 (Subsequence 3) = caggcgtttgttggttgattaa (22 bp).

The following three longest and second longest conserved subsequences are obtained, with a length of 22 bp to 30 bp, which are comparable to a length of small RNAs, but these three conserved subsequences are not included in higher organisms, especially human beings:
SEQ ID NO: 4 (Subsequence 1) = gttttacgacaacgatgttggtttaggaca (30 bp);
SEQ ID NO: 5 (Subsequence 2) = ggttcggttgttatatacgata (22 bp);
SEQ ID NO: 6 (Subsequence 3) = ggttcagagagtctcctattta (22 bp).

The following five conserved microsatellite loci with repeated nucleotides are obtained, where microsatellites are CTCTCT, AGAGAG, AAAAAAA, TATATA, and CACACA, respectively:

### 2. Design of common target siRNA for COVID-19 virus variant strains

A whole genome sequence (cDNA) of β-coronavirus (especially the COVID-19 virus and its variant strains) is downloaded from the Genbank database (http://www.NCBI.nlm.nih.gov/genome/); with Ambion's shRNA online design software (http://www.ambion.com/techlib/misc/siRNAtools.html) or DSIR and the like, multiple siRNA candidates are obtained with a length of about 19 nt; based on a Tm value of RNA binding and specificity alignment results, the siRNA is preferably selected. Therefore, RNAi sequences (siRNAs) of each strain and common RNAi sequences (common target siRNAs) of each strain are obtained from the *E* gene, *M* gene, *N* gene, *ORF1ab* gene, and S gene of 18 COVID-19 virus strains and their variant strains that are discovered so far. The common siRNAs of each strain are shown in Table 1, with sequences marked as ***SEQ ID NO: 7 to SEQ ID NO: 40.*** For example, the siRNAs (***SEQ ID NO: 41 to SEQ ID NO: 58***) marked in Tables 2 to 5 are the common target siRNAs of NC_045512.2, Delta strain, and Omicron strain, and the siRNAs without marking are their respective RNAi sequences (siRNAs). It is seen that although the earliest NC_045512.2 strain has mutated into the Delta strain and the recent Omicron strain, each strain, except for its own unique targeting interference sequence siRNA (parts without marking), still remains unchanged and theoretically has common conserved sequences ***SEQ ID NO: 41 to SEQ ID NO:* 58** with a targeted interference effect.

**Table 1 Common target siRNAs (SEQ ID NO: 7 to SEQ ID NO: 40) screened from 18 COVID-19 virus variant strains**

| Variant strain | Region | Position | Common target siRNA (ss sequence) of variant strains | Score | Off-targets | SEQ ID NO: |
|---|---|---|---|---|---|---|
| (1) B.1.617.1, (2) B.1.1.529, (3) B.1.1.7, (4) P.1, (5) BA.2, (6) B.1.351, (7) B.1.525, (8) B.1.617.2, (9) C.1.2, (10) B.1.621.1, (11) B.1.621, (12) P.2, (13) N.9, (14) C.37.1, (15) C.37, (16) B.1.427, (17) B.1.351.2, (18) B.1.351.3 | *E* gene | 7 | **gtggtattcttgctagttaca** | **95.1** | **0** | **SEQ ID NO: 7** |
| | *M* gene | 35 | **ggatttgtcttctacaatttg** | **95.7** | **0** | **SEQ ID NO: 8** |
| | | 146 | **cgaacgctttcttattacaaa** | 94.8 | **0** | SEQ ID NO: 9 |
| | | 130 | **cactgttgctacatcacgaac** | 94.8 | **0** | SEQ ID NO: 10 |
| | | 6 | **cctagtaataggtttcctatt** | 94.7 | 0 | SEQ ID NO: 11 |
| | | 181 | **gcgtgtagcaggtgactcagg** | 94.3 | 0 | SEQ ID NO: 12 |
| | | 18 | gaccgcttctagaaagtgaac | 93.3 | 0 | SEQ ID NO: 13 |
| | | 109 | caaggacctgcctaaagaaat | 91 | 0 | SEQ ID NO: 14 |
| | | 33 | atggatttgtcttctacaatt | 91.0 | 0 | SEQ ID NO: 15 |
| | *N* gene | 137 | **cgtagtcgcaacagttcaaga** | **100.7** | **0** | **SEQ ID NO: 16** |
| | | 12 | **ggtggaccctcagattcaact** | **99.9** | **0** | **SEQ ID NO: 17** |
| | | 56 | **ggttcaccgctctcactcaac** | **91.4** | **0** | **SEQ ID NO: 18** |
| | | 39 | cgggaacgtggttgacctaca | 90.4 | 0 | SEQ ID NO: 19 |
| | *ORFlab* gene | 7687 | **gcttatgtgtcaacctatact** | **106.6** | **0** | **SEQ ID NO: 20** |
| | | 8304 | **ggttgaagcagttaattaaag** | **105.1** | **0** | **SEQ ID NO: 21** |
| | | 8613 | **cgatattacgcacaactaatg** | **104.6** | **0** | **SEQ ID NO: 22** |
| | | 10519 | agaccatgttgacatactagg | 104.4 | 0 | SEQ ID NO: 23 |
| | | 6719 | ggttctttaatctactcaacc | 104.4 | 0 | SEQ ID NO: 24 |
| | | 1805 | ggtggtgttgttcagttgact | 104.3 | 0 | SEQ ID NO: 25 |
| | | 1619 | gctcgtgttgtacgatcaatt | 104.2 | 0 | SEQ ID NO: 26 |
| | | 11328 | agtgtataatgctagtttatt | 103.9 | 0 | SEQ ID NO: 27 |
| | | 4932 | ggtacatgtcagcattaaatc | 103.8 | 0 | SEQ ID NO: 28 |
| | | 2935 | gttagatgatgatagtcaaca | 103.5 | 0 | SEQ ID NO: 29 |
| | *S* gene | 4 | **gattgtttaggaagtctaatc** | **103.6** | **0** | **SEQ ID NO: 30** |
| | | 29 | **gtctctagtcagtgtgttaat** | **101.5** | **0** | **SEQ ID NO: 31** |
| | | 33 | **ctactaatgttgttattaaag** | **99.8** | **0** | **SEQ ID NO: 32** |
| | | 6 | ggtgttcttactgagtctaac | 99.2 | 0 | SEQ ID NO: 33 |
| | | 68 | gaaacaaagtgtacgttgaaa | 97.1 | 0 | SEQ ID NO: 34 |
| | | 155 | gttagatttcctaatattaca | 96.7 | 0 | SEQ ID NO: 35 |
| | | 7 | ctgtgatgttgtaataggaat | 94.9 | 0 | SEQ ID NO: 36 |
| | | 271 | ggagtcaaattacattacaca | 94.6 | 0 | SEQ ID NO: 37 |
| | | 12 | gettactctaataactctatt | 94.6 | 0 | SEQ ID NO: 38 |
| | | 2 | ggtaactgtgatgttgtaata | 94.1 | 0 | SEQ ID NO: 39 |
| | | 1 | tggtaactgtgatgttgtaat | 94.1 | 0 | SEQ ID NO: 40 |

**Table 2 siRNA candidate sequences of E gene of NC_045512.2, DELTA and OMICRON strains of COVID-19 virus**

| siRNA ID (*E*) | | Position | ss sequence (siRNA) | Score | Off-targets | SEQ ID NO: |
|---|---|---|---|---|---|---|
| NC_045512.2 | 1 | 16 | ggaagagacaggtacgttaat | 101.7 | 0 | SEQ ID NO: 62 |
| | 2 | 17 | gaagagacaggtacgttaata | 98 | 0 | SEQ ID NO: 63 |
| | ***3*** | ***26*** | ***ggtacgttaatagttaatage*** | **96.4** | ***0*** | ***SEQ ID NO: 41*** |
| | ***5*** | ***68*** | ***gtggtattcttgctagttaca*** | **95.1** | ***0*** | ***SEQ ID NO: 42*** |
| | ***6*** | ***31*** | ***gttaatagttaatagcgtact*** | **95** | ***0*** | ***SEQ ID NO: 43*** |
| DELTA | 1 | 16 | ggaagagacaggtacgttaat | 101.7 | 0 | SEQ ID NO: 62 |
| | 2 | 17 | gaagagacaggtacgttaata | 98 | 0 | SEQ ID NO: 63 |
| | ***3*** | ***26*** | ***ggtacgttaatagttaatage*** | **96.4** | ***0*** | ***SEQ ID NO: 41*** |
| | ***5*** | ***68*** | ***gtggtattcttgctagttaca*** | **95.1** | ***0*** | ***SEQ ID NO: 42*** |
| | ***6*** | ***31*** | ***gttaatagttaatagcgtact*** | **95** | ***0*** | ***SEQ ID NO: 43*** |
| OMICRON | 1 | 16 | ggaagagataggtacgttaat | 102.3 | 0 | SEQ ID NO: 62 |
| | 2 | 17 | gaagagataggtacgttaata | 98.6 | 0 | SEQ ID NO: 63 |
| | ***3*** | ***68*** | ***gtggtattcttgctagttaca*** | **95.1** | ***0*** | ***SEQ ID NO: 42*** |
| | ***4*** | ***31*** | ***gttaatagttaatagcgtact*** | **95** | ***0*** | ***SEQ ID NO: 43*** |
| | ***5*** | ***26*** | ***ggtacgttaatagttaatage*** | **94.9** | **1** | ***SEQ ID NO: 41*** |

**Table 3 siRNA candidate sequences of M gene of NC_045512.2, DELTA and OMICRON strains of COVID-19 virus**

| siRNA ID (*M*) | | Position | ss sequence(sirna) | Score | Off-targets | SEQ ID NO: |
|---|---|---|---|---|---|---|
| NC_045512.2 | 1 | 200 | gctgctgtttacagaataaat | 103.4 | 0 | SEQ ID NO: 64 |
| | 2 | 13 | eggtactattaccgttgaaga | 97.4 | 0 | SEQ ID NO: 65 |
| | 3 | ***230*** | ***ggtggaattgctatcgcaatg*** | **97.2** | ***0*** | ***SEQ ID NO: 44*** |
| | 4 | ***90*** | ***ggatttgtcttctacaatttg*** | **95.7** | ***0*** | ***SEQ ID NO: 45*** |
| | 5 | ***518*** | ***cgaacgctttcttattacaaa*** | ***94.8*** | ***0*** | ***SEQ ID NO: 46*** |
| | 6 | ***502*** | ***cactgttgctacatcacgaac*** | ***94.8*** | ***0*** | ***SEQ ID NO: 47*** |
| | 9 | **61** | ***cctagtaataggtttcctatt*** | **94.7** | ***0*** | ***SEQ ID NO: 48*** |
| DELTA | 1 | 200 | gctgctgtttacagaataaat | 103.4 | 9 | SEQ ID NO: 64 |
| | 2 | 13 | cggtactattaccgttgaaga | 97.4 | 1 | SEQ ID NO: 65 |
| | ***3*** | ***230*** | ***ggtggaattgctatcgcaatg*** | **97.2** | ***0*** | ***SEQ ID NO: 44*** |
| | ***4*** | ***90*** | ***ggatttgtcttctacaatttg*** | **95.7** | ***0*** | ***SEQ ID NO: 45*** |
| | ***5*** | ***518*** | ***cgaacgctttcttattacaaa*** | ***94.8*** | ***0*** | ***SEQ ID NO: 46*** |
| | ***6*** | ***502*** | ***cactgttgctacatcacgaac*** | ***94.8*** | ***0*** | ***SEQ ID NO: 47*** |
| | ***9*** | ***61*** | ***cctagtaataggtttcctatt*** | **94.7** | ***0*** | ***SEQ ID NO: 48*** |
| OMICRON | 1 | 200 | gctgctgtttacagaataaat | 103.4 | 9 | SEQ ID NO: 64 |
| | 2 | 5 | ggttccaacggtactattacc | 99.5 | 0 | SEQ ID NO: 66 |
| | 3 | 13 | eggtactattaccgttgaaga | 97.4 | 1 | SEQ ID NO: 65 |
| | ***4*** | ***230*** | ***ggtggaattgctatcgcaatg*** | **97.2** | ***0*** | ***SEQ ID NO: 44*** |
| | ***5*** | ***90*** | ***ggatttgtcttctacaatttg*** | **95.7** | ***0*** | ***SEQ ID NO: 45*** |
| | ***6*** | ***518*** | ***cgaacgctttcttattacaaa*** | ***94.8*** | ***0*** | ***SEQ ID NO: 46*** |
| | ***7*** | ***502*** | ***cactgttgctacatcacgaac*** | ***94.8*** | ***0*** | ***SEQ ID NO: 47*** |
| | ***9*** | **61** | ***cctagtaataggtttcctatt*** | **94.7** | ***0*** | ***SEQ ID NO: 48*** |

**Table 4 siRNA candidate sequences of N gene of NC_045512.2, DELTA and OMICRON strains of COVID-19 virus**

| siRNA ID (*N*) | | Position | ss sequence(sirna) | Score | Off-targets | SEQ ID NO: |
|---|---|---|---|---|---|---|
| NC_045512.2 | 1 | 260 | cgaagagctaccagacgaatt | 101.1 | 1 | SEQ ID NO: 67 |
| | 2 | 1150 | gagacagaagaaacagcaaac | 100.1 | 36 | SEQ ID NO: 68 |
| | ***3*** | ***563*** | ***cgtagtcgcaacagttcaaga*** | ***100.7*** | ***0*** | ***SEQ ID NO: 49*** |
| | 4 | 1198 | ggatgatttctccaaacaatt | 100.2 | 12 | SEQ ID NO: 69 |
| | 5 | 50 | ggtggaccctcagattcaact | 99.9 | 1 | SEQ ID NO: 17 |
| | ***6*** | **251** | ***ggctactaccgaagagctacc*** | **99.9** | ***0*** | ***SEQ ID NO: 50*** |
| | ***14*** | ***306*** | ***caagatggtatttctactacc*** | ***95.6*** | ***0*** | ***SEQ ID NO: 51*** |
| DELTA | 1 | 260 | cgaagagctaccagacgaatt | 101.1 | 1 | SEQ ID NO: 67 |
| | 2 | 1150 | gagacagaagaaacagcaaac | 101.1 | 36 | SEQ ID NO: 68 |
| | ***3*** | ***563*** | ***cgtagtcgcaacagttcaaga*** | ***100.7*** | ***0*** | ***SEQ ID NO: 49*** |
| | 4 | 1198 | ggatgatttctccaaacaatt | 100.2 | 12 | SEQ ID NO: 69 |
| | 5 | 50 | ggtggaccctcagattcaact | 99.9 | 1 | SEQ ID NO: 17 |
| | ***6*** | **251** | ***ggctactaccgaagagctacc*** | **99.9** | ***0*** | ***SEQ ID NO: 50*** |
| | ***14*** | ***315*** | ***caagatggtatttctactacc*** | ***95.6*** | ***0*** | ***SEQ ID NO: 51*** |
| OMICRON | 1 | 251 | cgaagagctaccagacgaatt | 101.1 | 1 | SEQ ID NO: 67 |
| | 2 | 1141 | gagacagaagaaacagcaaac | 101.1 | 36 | SEQ ID NO: 68 |
| | ***3*** | ***554*** | ***cgtagtcgcaacagttcaaga*** | ***100. 7*** | ***0*** | ***SEQ ID NO: 49*** |
| | 4 | 1189 | ggatgatttctccaaacaatt | 100.2 | 12 | SEQ ID NO: 69 |
| | 5 | 50 | ggtggaccctcagattcaact | 99.9 | 1 | SEQ ID NO: 17 |
| | ***6*** | **242** | ***ggctactaccgaagagctacc*** | **99.9** | ***0*** | ***SEQ ID NO: 50*** |
| | ***14*** | ***306*** | ***caagatggtatttctactacc*** | ***95.6*** | ***0*** | ***SEQ ID NO: 51*** |

**Table 5 siRNA candidate sequences of ORF1ab gene of NC_045512.2, DELTA and OMICRON strains of COVID-19 virus**

| siRNA ID (*ORF*) | | Position | ss sequence(sirna) | Score | Off-targets | SEQ ID NO: |
|---|---|---|---|---|---|---|
| NC_045512.2 | 1 | 13351 | ggacgaagatgacaatttaat | 111.5 | 0 | SEQ ID NO: 70 |
| | 2 | 5654 | ggtgttgtttgtacagaaatt | 107.7 | 0 | SEQ ID NO: 71 |
| | 3 | 17851 | cctcagtgttgacactaaatt | 107.2 | 0 | SEQ ID NO: 72 |
| | ***4*** | ***7687*** | ***gcttatgtgtcaacctatact*** | ***106.6*** | ***0*** | ***SEQ ID NO: 52*** |
| | ***9*** | ***8304*** | ***ggttgaagcagttaattaaag*** | ***105.1*** | ***0*** | ***SEQ ID NO: 53*** |
| | ***13*** | ***8610*** | ***cgatattacgcacaactaatg*** | ***104.6*** | ***0*** | ***SEQ ID NO: 54*** |
| DELTA | 1 | 13351 | ggacgaagatgacaatttaat | 111.5 | 7 | SEQ ID NO: 70 |
| | 2 | 5654 | ggtgttgtttgtacagaaatt | 107.7 | 3 | SEQ ID NO: 71 |
| | 3 | 17851 | cctcagtgttgacactaaatt | 107.2 | 6 | SEQ ID NO: 72 |
| | ***4*** | ***7687*** | ***gcttatgtgtcaacctatact*** | ***106.6*** | ***0*** | ***SEQ ID NO: 52*** |
| | ***9*** | ***8304*** | ***ggttgaagcagttaattaaag*** | ***105.1*** | ***0*** | ***SEQ ID NO: 53*** |
| | ***13*** | ***8613*** | ***cgatattacgcacaactaatg*** | ***104.6*** | ***0*** | ***SEQ ID NO: 54*** |
| OMICRON | 1 | 13339 | ggacgaagatgacaatttaat | 111.5 | 7 | SEQ ID NO: 70 |
| | 2 | 5654 | ggtgttgtttgtacagaaatt | 107.7 | 3 | SEQ ID NO: 71 |
| | 3 | 17839 | cctcagtgttgacactaaatt | 107.2 | 6 | SEQ ID NO: 72 |
| | ***4*** | ***7684*** | ***gcttatgtgtcaacctatact*** | ***106.6*** | ***0*** | ***SEQ ID NO: 52*** |
| | ***9*** | ***8301*** | ***ggttgaagcagttaattaaag*** | ***105.1*** | ***0*** | ***SEQ ID NO: 53*** |
| | ***13*** | ***8610*** | ***cgatattacgcacaactaatg*** | ***104.6*** | ***0*** | ***SEQ ID NO: 54*** |

**Table 6 siRNA candidate sequences of S gene of NC_045512.2, DELTA and OMICRON strains of COVID-19 virus**

| siRNA ID *(S)* | | Position | ss sequence(sirna) | Score | Off-targets | SEQ ID NO: |
|---|---|---|---|---|---|---|
| NC_045512.2 | 1 | 1837 | ggatgttaactgcacagaagt | 105.8 | 0 | SEQ ID NO: 73 |
| | ***2*** | ***1359*** | ***gattgtttaggaagtctaatc*** | ***103.6*** | ***0*** | ***SEQ ID NO: 55*** |
| | 3 | 1074 | gcaactgtgttgctgattatt | 101.9 | 0 | SEQ ID NO: 74 |
| | ***4*** | ***29*** | ***gtctctagtcagtgtgttaat*** | ***101.5*** | ***0*** | ***SEQ ID NO: 56*** |
| | ***8*** | ***366*** | ***ctactaatgttgttattaaag*** | ***99.8*** | ***0*** | ***SEQ ID NO: 57*** |
| | ***13*** | ***1646*** | ***ggtgttcttactgagtctaac*** | **99.2** | ***0*** | ***SEQ ID NO: 58*** |
| DELTA | ***1*** | ***1359*** | ***gattgtttaggaagtctaatc*** | ***103.6*** | ***0*** | ***SEQ ID NO: 55*** |
| | 2 | 1837 | gggtgttaactgcacagaagt | 102.4 | 0 | SEQ ID NO: 75 |
| | 3 | 1353 | ggtatagattgtttaggaagt | 102 | 0 | SEQ ID NO: 76 |
| | ***5*** | ***29*** | ***gtctctagtcagtgtgttaat*** | ***101.5*** | ***0*** | ***SEQ ID NO: 56*** |
| | ***9*** | ***366*** | ***ctactaatgttgttattaaag*** | ***99.8*** | ***0*** | ***SEQ ID NO: 57*** |
| | ***13*** | ***1646*** | ***ggtgttcttactgagtctaac*** | **99.2** | ***0*** | ***SEQ ID NO: 58*** |
| OMICRON | ***1*** | ***1350*** | ***gattgtttaggaagtctaatc*** | ***103.6*** | ***0*** | ***SEQ ID NO: 55*** |
| | 2 | 1828 | gggtgttaactgcacagaagt | 102.4 | 0 | SEQ ID NO: 75 |
| | 3 | 1065 | gcaactgtgttgctgattatt | 101.9 | 1 | SEQ ID NO: 74 |
| | ***4*** | ***29*** | ***gtctctagtcagtgtgttaat*** | ***101.5*** | ***0*** | ***SEQ ID NO: 56*** |
| | ***8*** | ***360*** | ***ctactaatgttgttattaaag*** | ***99.8*** | ***0*** | ***SEQ ID NO: 57*** |
| | ***12*** | ***1637*** | ***ggtgttcttactgagtctaac*** | **99.2** | ***0*** | ***SEQ ID NO: 58*** |

### 3. Screening of siRNA by targeting ultra-conserved genes, conserved genes, or conserved microsatellites

With the Clustal W software or other software, gene sequence alignment is conducted on the ultra-conserved genes, conserved genes, and conserved microsatellites with conventionally-screened siRNAs, to detect a similarity between different sequences; multiple pairs of siRNAs are designed, which are the ultra-conserved genes, conserved genes, or conserved microsatellites, as well as RNAi target sites (siRNAs by targeting ultra-conserved genes, conserved genes, or conserved microsatellites are designed).
**(1) siRNA by targeting ultra-conserved genes and conserved microsatellites (S1/S2):**
   SEQ ID NO: 1 (Subsequence 1) = ttaatacgacctctctgttggattttgaca (30 bp);
   SEQ ID NO: 2 (Subsequence 2) = ggttcgcaacttcacaca gagt (22 bp);
**(2) siRNA by targeting conserved genes and conserved microsatellites (S3/S4):**
   SEQ ID NO: 5 (Subsequence 3) = ggttcggttgttatatacgata (22 bp);
   SEQ ID NO: 6 (Subsequence 4) = ggttcagagagtctcctattta (22 bp).

Through the above design, siRNAs that theoretically resist coronavirus variant strains are obtained with ultra-conserved genes, conserved genes, or conserved microsatellites as interference targets, named siRNA1/2/3/4.

### II. Validation of common target siRNAs

### 1. Synthesis of siRNA/shRNA

According to the RNAi mechanism, when siRNA effectively interferes with the mRNA expression of the *S* gene, an S protein-deficient virus that loses infectivity is formed. When the siRNA effectively interferes with the mRNA expression of the *N* gene, the packaging and replication of the virus may be inhibited. When siRNA effectively interferes with the mRNA expression of *ORF1a* or *ORF1b* gene, the synthesis of viral RNA polymerase (RdRp) or protein processing enzyme (3CLpro) may be affected. However, the *M* and *E* genes are membrane genes of the virus, with an inhibitory effect of their gene defects on the virus being not obvious. Therefore, siRNA targeting N gene (SEQ ID NO: 16 to SEQ ID NO: 18, SEQ ID NO: 49 to SEQ ID NO: 51), siRNA targeting *ORF1ab* gene (SEQ ID NO: 20 to SEQ ID NO: 22, SEQ ID NO: 52 to SEQ ID NO: 54), and siRNA targeting S gene (SEQ ID NO: 30 to SEQ ID NO: 32, SEQ ID NO: 56 to SEQ ID NO: 58), and SEQ ID NO: 1 to SEQ ID NO: 2 and SEQ ID NO: 5 to SEQ ID NO: 6 are selected for synthesis. In addition, according to polyclonal restriction enzyme cleavage site of a pSilencer4.1.CMVneo interference vector, a shRNA template capable of expressing a hairpin structure is designed; each template is composed of two mostly complementary 55 bp single-stranded DNAs, and the single-stranded DNA can be annealed and complementary to form a double-stranded DNA with sticky ends of *Bam* HI and *Hind* III restriction sites for ligation with a linearized pSilencer4.1.CMV.neo. According to the designed siRNA and its shRNA template, a company is commissioned to synthesize the siRNA.

### 2. Construction of shRNA expression vector

The shRNA is ligated with the linearized interference vector pSilencer4.1.CMV.neo, and then identified to construct a shRNA expression plasmid, and transformed into DH5a to obtain the shRNA expression vector.

### 3. Identification of effect of shRNA expression (interference) vector

According to the synthesized siRNA/shRNA and its constructed expression plasmid, a corresponding target gene is selected for synthesis or PCR amplification, a fluorescent tag vector is constructed, and co-transfected into type II alveolar epithelial cells (AEC2s) or 293T cells separately with the shRNA expression plasmid, and the cells are identified. A conventional method of PCR amplification is as follows:

**Primer design:** upstream and downstream primers are designed, a start codon is added at a 5'-end of the upstream primer, and a homology arm is added to the 5'-end of the primer for homologous recombination with a vector in order to clone an amplification product into pEGFP-N1.

**Target gene amplification:** gene amplification, product recovery, and purification are conducted according to a gene amplification reaction system and reaction conditions provided by a Shanghai Sangon kit to obtain the amplification product.

**Linearization of pEGFP-N1:** a DH5a strain containing a pEGFP-N1 plasmid is resuscitated, the plasmid is extracted by the kit, the concentration is determined, restriction digestion is conducted, and a linearized vector is identified and recovered by 0.8% agarose gel electrophoresis.

**An amplified target gene is ligated to a fluorescent tag vector (pEGFP-N1):** the ligation is conducted with a GenScript's homologous recombination kit, and a ligated product can be stored at -20°C for later use or transformed immediately.

**Identification of effects of a shRNA interference vector:** the interference vector (pSilencer-shRNA) and the fluorescent tag vector (pEGFP-N/S/ORF1ab) are co-transfected into 293T cells, where the interference vector and the tag vector have a mass ratio of 1:2, while a control is set up; the fusion expression of a GFP protein in the cells is observed 48 h after the transfection, and an interference effect is evaluated according to a fluorescence intensity.

**Flow cytometry:** to quantitatively analyze the interference effects of different interference vectors, flow cytometry is conducted to analyze a proportion of fluorescent protein-expressing cells in the total number of cells.

**Westernbolt analysis:** (1) cell collection and lysis: cells are lysed with RIPA; (2) SDS-PAGE protein electrophoresis: an SDS-PAGE gel is prepared, a sample is added to an equal volume of a 2×SDS buffer, boiled in boiling water for 5 min, treated in an ice bath for 2 min, and then centrifuged at 12,000×g for 10 min; (3) Western blot detection: after conducting transferring, blocking, primary antibody binding, washing, secondary antibody binding, and color development, results are observed.

**RT-PCR detection of mRNA:** relative fluorescence quantitative RT-PCR is conducted to detect a relative expression level of the target genes in transfected cells; according to a standard curve, a copy number of the target gene and a B-actin reference gene is converted from a CT value; a relative expression level of the viral gene mRNA (the number of copies of the target gene/the number of copies of the B-actin) is corrected with the B-actin reference gene, such that an interference effect is quantitatively evaluated.

### 4. Obtaining siRNA/shRNA with a high silencing efficiency

After design, synthesis, screening, iterative design, resynthesis, and verification at the cellular level, siRNAs with a high silencing efficiency are obtained. The siRNAs have sequences of SEQ ID NO: 1 (named shRNA1, the same below), SEQ ID NO: 2 (shRNA2), SEQ ID NO: 5 (shRNA3), SEQ ID NO: 16 (shRNA4) and SEQ ID NO: 49 (shRNA5) targeting the N gene, SEQ ID NO: 21 (shRNA6) and SEQ ID NO: 52 (shRNA7) targeting the *ORF1ab* gene, and SEQ ID NO: 30 (shRNA8) targeting the S gene, with silencing efficiencies of 78%, 76%, 88%, 89%, 89%, 84%, 91%, and 90%, respectively.

### III. Synthesis of shRNA

According to the common targets (SEQ ID NO: 1 to SEQ ID NO: 58), SEQ ID NO: 5 (shRNA3), SEQ ID NO: 49 (shRNA5), and SEQ ID NO: 30 (shRNA8) with a shorter target gene sequence are selected, synthesized by a biological company. Each shRNA is synthesized with 2 complementary oligonucleotide siRNAs of 19 nt to 25 nt, and synthesizes a 9 nt base sequence that acts as a spacer; the siRNA (drug) and the base sequence are ligated into a double-stranded small hairpin shRNA with a loop formed by separation of intermediate base sequences; each single strand of the double-stranded shRNA can be ligated to an ACE2 polypeptide or protein, respectively.

For example, SEQ ID NO: 1 (shRNA1), SEQ ID NO: 2 (shRNA2), and SEQ ID NO: 5 (shRNA3) are synthesized into 5'-ttaatacgacctctctgttggattttgaca**ttcaagaga**tgtcaaatccaacagagaggtcgtattaa-3' (shRNA1) (SEQ ID NO: 77), 5'-ggttcgcaacttcacacagagt**ttcaagaga**actctgtgtgaagttgcgaacc-3' ( shRNA2) (SEQ ID NO: 78) and 5'-ggtt cggt tgtta tatac gata **ttcaagaga** tatc gtata taaca accg aacc-3' (shRNA3) (SEQ ID NO: 79), where "**TTCAAGAGA**" is a loop, and left and right sides thereof are complementary sense and antisense strands, and then a 3'-and/or 5'-end of the shRNA is ligated with the ACE2 protein or polypeptide separately. Similarly, preferably other siRNAs with a high silencing efficiency are synthesized into shRNAs.

### IV. Design and synthesis of targeted delivery vector ACE2

### 1. Amino acid sequences of ACE2

The human ACE2 gene sequence information was searched from the GeneBank database (http://www.ncbi.nlm.gov/genbank); the ACE2 consists of 805 amino acids, where amino acids 1 to 740 (SEQ ID NO: 59) are located extracellularly, amino acids 741 to 763 (SEQ ID NO: 60) are located in a transmembrane region, and amino acids 764 to 805 (SEQ ID NO: 61) are located intracellularly. Among the 20 amino acids that make up the ACE2 protein, leucine accounts for 9.4%, cysteine and histidine account for 1.0% and 2.0%, respectively, and negatively-charged amino acid residues (aspartate + glutamate) and positively-charged amino acid residues (arginine + lysine) as a balance. SARS-CoV and SARS-CoV 2 interact with an extracellular catalytic domain of the ACE2 through virus RBD. This interaction can lead to endocytosis and membrane fusion, such that the SARS-CoV enters cells that highly express ACE2 or contain ACE2 receptors.

### 2. Receptor function of ACE2

ACE2 is a lipid-soluble type I transmembrane glycoprotein with an amino-terminal catalytic domain and a carboxyl-terminal domain. The N-terminus is outside the cell membrane and the C-terminus is inside the cell membrane. This glycoprotein is divided into an N-terminal signal peptide region, a carboxypeptidase activation region, and a transmembrane region. When the Spike protein of coronavirus contacts a tip of a subdomain I of the catalytic domain of ACE2 (without affecting a subdomain II and a peptidase activity), an outer domain of the ACE2 is cleaved and the transmembrane domain is internalized, enabling further virus-host cell fusion. Therefore, it is believed that the transmembrane region is involved in the transport of virus-receptor complexes from the cell membrane to the cytoplasm.

### 3. Design of ACE2 of the present disclosure

From an amino acid sequence of ACE2 and a receptor function of ACE2, it can be seen that there are ACE2 receptor channels in the cell wall and cell membrane of ACE2-expressing cells. Full-length ACE2, transmembrane region ACE2, intracellular ACE2, and extracellular ACE2 each are a membrane-penetrating polypeptide with a function of targeted delivery of siRNA. The N-terminus of extracellular ACE2 has a function of neutralizing virus by binding to the coronavirus RBD. Therefore, full-length ACE2, extracellular ACE2 of amino acids 1 to 740, transmembrane ACE2 of amino acids 741 to 763, or intracellular ACE2 of amino acids 764 to 805, or ACE2 with optimized amino acid sequences can be designed and synthesized as a targeted delivery vector for siRNA.

### 4. Synthesis of ACE2 of the present disclosure

Two amino acids are dehydrated and condensed to form peptide bonds, and multiple amino acid residues are ligated by the peptide bonds to form polypeptides. A company can be entrusted to automatically synthesize peptides by a peptide synthesizer. Basically, amino acids are added in order according to a sequence of the polypeptide to be synthesized, such that the peptide chain is gradually extended from the C-terminal to the N-terminal residues; each amino acid residue is required to be condensed in the form of protection at one end and activation at the other end, and temporary protection groups on the amino group are removed after each round of peptide chain elongation, until all amino acid sequences of the target polypeptide are condensed. At present, a commonly used reaction for solid-phase synthesis of the polypeptides includes: in a closed explosion-proof glass reactor, amino acids are continuously added from the C-terminus-carboxy terminus to the N-terminus-amino terminus according to a known sequence, and synthesis is conducted to finally obtain the polypeptides. A synthesis method includes: (1) deprotection: removing the protective group of the amino group with an alkaline solvent; (2) activation and cross-linking: activating a carboxyl group of a next amino acid, cross-linking an activated single carboxyl group with a free amino group to form a peptide bond; and repeating these two steps until the polypeptide is synthesized.

### V. Synthesis of nCoVshRNA·2ACE2 with ACE2 and shRNA

### 1. Design of nCoVshRNA-2ACE2

Take the synthesis of shRNA and extracellular, transmembrane or intracellular ACE2 as an example: one end of the sense and antisense strands of the synthesized siRNA/shRNA is ligated to the loop (5'-TTCAAGAGA-3'), and the other end is ligated to ACE2 (C-terminus), so as to obtain a structure of "ACE2-siRNA sense strand-loop-siRNA antisense strand-ACE2", which is expressed as "2ACE2-shRNA". The two complementary sense and antisense strands can form a double strand, but the two ACE2 polypeptides cannot form the double strand. As shown in FIG. 2, there is a hairpin ligation product shRNA with two ACE2 polypeptides, two siRNA sense and antisense strands, and a loop. Since the virus RBD infects cells through the C-terminus binding to the N-terminus of ACE2, and the binding of ACE2 polypeptide to siRNA can increase the permeability, stability and interference effect of siRNA. Accordingly, as shown in FIG. 3, ACE2 in this design can neutralize the virus and prevent virus infection, and can deliver siRNA/shRNA to the virus RBD in a targeted manner to form a complex of "shRNA-ACE2-RBD-virus". As a result, the shRNA is delivered by the virus, and the shRNA enters the target cells with virus infection, playing a role of targeted interference.

### 2. Synthesis of ACE2-shRNA-ACE2 (2ACE2-shRNA)

According to the design (FIG. 2), the entrusted company adopts the sequences of extracellular ACE2, SEQ ID NO: 5 (shRNA3), transmembrane ACE2, SEQ ID NO: 49 (shRNA5), and SEQ ID NO: 30 (shRNA8), by a conventional synthesis method of oligonucleotide, the polypeptide (ACE2) and oligonucleotide (shRNA/siRNA) are coupled to form a conjugate with a carboxyhydrazone bond, a disulfide bond, a phosphodiester bond, a phosphorodithioate bond, a thioether bond, an oxime bond, an amide bond, and a maleimide-thiol bond. The sense strand (5'-end and 3'-end) or antisense strand (3'-end) of polypeptides and oligonucleotides can be non-covalently or covalently cross-linked with a firmer covalent bond, a looser ionic bond, a hydrophobic bond, or the carboxyhydrazone bonds with a spacer arm to synthesize a polypeptide-oligonucleotide conjugate (POCs). At present, the POCs are generally synthesized by covalent crosslinking-liquid phase fragment synthesis, and various POCs are prepared. The method includes the following steps: synthesizing a polypeptide and an oligonucleotide separately on a solid-phase substrate, simultaneously peeling the polypeptide and the oligonucleotide from the solid-phase substrate, and coupling peeled polypeptide and oligonucleotide in a solution by a reactive group. Synthesis of POCs mainly includes: (1) Maleimide-thiol bond coupling: maleimide is modified on the polypeptide or oligonucleotide, thiol is modified on another monomer, and the two monomers are added into a same solution to obtain the POCs after a reaction. (2) Disulfide bond or thioether bond coupling: 5'- or 3'-positions of the oligonucleotide is modified with a thiol group, and then reacted with a polypeptide whose C-terminus is modified with a bromoacetyl group in a buffer solution of pH 7.0; the disulfide bond coupling can be directly oxidized by two thiol groups, or the thiol group can be activated by an activator such as dipyridyl disulfide and then coupled with another oligomer containing a thiol group; the disulfide bonds are commonly used to synthesize a conjugate of siRNA and polypeptides. (3) Oxime bond coupling: the aldehyde group reacts with the amino group to produce oxime; the reaction conditions are mild, with a high reaction efficiency, and a coupling product of double-stranded DNA and a specific polypeptide can be directly generated; meanwhile, two polypeptides can be simultaneously ligated to the 5'- and 3'-end of the nucleic acid through an oxime bond, by a bifunctional oligonucleotide with a polypeptide or a carbohydrate. This method does not require various protection processes and can be completed in one step, which is used to synthesize a "peptide-oligonucleotide-peptide" product. Specifically, the aldehyde group is introduced into the 5'- and 3'-end of the oligonucleotide, and then reacted with a hydroxylamine-modified polypeptide to obtain a "peptide-oligonucleotide-peptide" with a high yield. This one-step reaction of bifunctionalized oligonucleotides with polypeptides does not require any protection strategies and cross-linking reagents, and has a high yield under the slightly acidic environment. (4) Amide bond coupling: an oligomer containing activated carboxylic acid or thioester is reacted with another polymer modified with an amino group to obtain a product. (5) Hydrazone bond coupling: a hydrazine group is introduced into the polypeptide, a citric acid buffer with a pH value of 3 to 5 is added, and the mixture is reacted with an oligonucleotide modified with an acetaldehyde group. Thus, POCs ligated by hydrazone bonds are obtained and expressed as "2ACE2-shRNA3", "2ACE2-shRNA5", and "2ACE2-shRNA8", respectively.

### 3. Purification of ACE2-shRNA- ACE2

Chromatographic methods are most commonly used for purification and analysis of the conjugate of peptides and oligonucleotides. According to complexity of the conjugates, different chromatographic methods should be selected for separation. The main methods include high-performance liquid chromatography (HPLC), reverse high-performance liquid chromatography (RP-HPLC), ion exchange chromatography (IEC, generally anion exchange chromatography), or two or more of which are used in series. Specifically, according to operating instructions, 2ACE2-shRNA3, 2ACE2-shRNA5, and 2ACE2-shRNA8 are extracted.

### 4. Synthesis of other compounds for delivery of shRNA by targeting ACE2

Similarly, shRNA (SEQ ID NO: 1 to SEQ ID NO: 58) can be ligated with intracellular ACE2 or codon-optimized ACE2 polypeptide to form compounds, including but not limited to "transmembrane ACE2-shRNA-transmembrane ACE2", and "intracellular ACE2 -shRNA-intracellular ACE2"; alternatively, the shRNA/siRNA is inserted into the middle of ACE2 polypeptide to synthesize "transmembrane ACE2-shRNA-extracellular ACE2", and "intracellular ACE2-shRNA-extracellular ACE2"; similarly, compounds with ACE2 or ACE2-optimized polypeptides as targeted delivery vectors are designed, including but not limited to "ACE2-siRNA, extracellular ACE2-siRNA, transmembrane ACE2-siRNA, and intracellular ACE2-siRNA".

### VI. Liposome modification of compounds

Liposome (Lip) modification includes: adsorption of positively-charged liposomes with negatively-charged shRNA, formation of the thiol-maleimide bond with thiol and liposomes by thiolation of amino groups in the ACE2 polypeptides, or formation of the carbamate bond with amino groups of ACE2 and liposomes (FIG. 4 to FIG. 5).

### Example 1 Preparation of liposome-modified compound with liposome DOTAP/Chol

### (1) Preparation of lipid solution

DOTAP (MW=698.5): 10 mg/ml, 100 mg of an N-1-(2,3-di-oleoyloxy)propyl)-N,N,N-trimethylammoniumethyl sulfate (DOTAP) powder was added into a 10 ml volumetric flask, and a chloroform solution was added to reach a scale line.

Chol (MW=386): 5 mg/ml, 50 mg of a cholesterol (Chol) powder was added into a 10 ml volumetric flask, and a chloroform solution was added to reach a scale line.

m-PEG2000-DSPE (MW=2787): 10 mg/ml, 10 mg of a methoxy-polyethylene glycol-distearoyl phosphatidyl-ethanolamine (m-PEG2000-DSPE) powder was added to 1 ml of DEPC water, and dissolved completely by vortexing and sonication for 1 min.

Mal-PEG2000-DSPE (MW=2941.6): 10 mg/ml, 10 mg of a maleimide derivatized polyethylene glycol-distearoyl phosphatidyl-ethanolamine (Mal-PEG2000-DSPE) powder was added to 1 ml of DEPC water, and dissolved by vortexing and sonication for 1 min.

### (2) Preparation of liposome DOTAP/Chol (lipid-film method)

The liposome DOTAP/Chol was prepared by a lipid-film method, at a lipid concentration of 10 mM. The method included the following steps: according to an amount of preparing 1 ml of the liposome DOTAP/Chol, 3 ml to 4 ml of chloroform solutions of DOTAP and Chol were separately added to a 500 ml conical flask at a ratio of DOTAP: Chol=1:1 (M:M); vacuum rotary evaporation was conducted at 37°C for 45 min to 60 min to obtain a uniform lipid film, and traces of chloroform were blown out with high-purity nitrogen; 1 ml of DEPC water was added to shake to wash the lipid film from a bottle wall, to obtain a lipid suspension; after being fully hydrated, the lipid film was sonicated for 1 min, and extruded through 400 nm, 200 nm, 80 nm, and 50 nm of polycarbonate membranes in sequence for 10 to 20 times in each size, so as to obtain the liposome DOTAP/Chol.

### (3) Thiolation of ACE2 and 2ACE2-shRNA

2-IT (Traut'S reagent) was a common reagent for protein thiolation, and the thiolation was conducted at an N amino group of the ACE2 protein. The thiolation included the following steps: 2ACE2-shRNA and 2-IT (Traut'S reagent, 2-iminothiolane-HCl) were mixed evenly (the 2-IT and the 2ACE2-shRNA have a molar ratio of 200: 1), and reacted at a room temperature for 2 h; excess 2-IT was removed by dialysis with a sufficient amount of the dialysate (1×PBS, 5 mM EDTA, pH=7.4) that was stored at 4°C for each time, where the dialysis was conducted overnight by magnetic stirring and the dialysate was changed after 6 h to 8 h; a protein concentration and a degree of thiolation were determined by a BCA method and an Ellman method, respectively.

### (4) Preparation of liposome-modified compound with liposome DOTAP/Chol

### (A) Preparation by negatively-charged siRNA/shRNA adsorbing positively-charged liposomes

(1) 120 µl of the DOTAP/Chol liposome (10 mM) was added with 20 µl of the 2ACE2-shRNA (about 2 µg/µl, 40 µg) and 11 µl of DEPC water, and allowed to stand for 10 min at a room temperature to obtain a liposome-modified complex 2ACE2-shRNA/ch1.
(2) 90 µl of siRNA (24 µg, 20 mM) or 90 µl of shRNA (24 µg, 10 mM) was added with 57.6 µl of DEPC water, allowed to at a room temperature for 10 min, and added with 600 µl of the DOTAP/Chol liposome (50 mM) to obtain siRNA/ch1 and shRNA/ch1.
(3) The liposome-modified complex 2ACE2-shRNA/ch1 prepared in (1) and the liposome-modified complex siRNA/ch or shRNA/ch prepared in (2) were mixed equivalently to obtain a corresponding mixed liposome-modified complex.

### (B) Preparation by cross-linking of thiol groups in ACE2 and maleimide in PEG

To increase a circulation time and targeting specificity of the liposomes, various liposome-modified complexes prepared in (A) were further PEGylated and ACE2-modified to obtain a PEGylated liposome-modified complex with RBD as a ligand.

9.53 µl (while setting 6.36 µl and 12.7 µl as a control) of 10 mg/ml MAL-DSPE-PEG was inserted into the liposome-modified complex 2ACE2-shRNA/ch1, siRNA/ch1, or shRNA/ch1 prepared in (A) separately (mixing the two separately), incubated in a 50°C water bath for 10 min, and allowed to stand for 10 min at a room temperature; about 200 µg of thiolated ACE2 or 2ACE2-shRNA/ch1 was added to cross-link the thiol group on the thiolated amino group in the ACE2 with the maleimide in MAL-DSPE-PEG, to obtain ACE2-modified and PEGylated (PEGylation had PEG concentrations of 5 mol% PEG, 7.5 mol% PEG, and 10 mol% PEG, respectively). That is, the complex was electrostatically adsorbed to the liposome DOTAP/Chol with siRNA, shRNA, and/or 2ACE2-shRNA, wrapped on an outer layer with the MAL-DSPE-PEG, and then ligated with the ACE2 or 2ACE2-shRNA by the MAL-DSPE-PEG. The prepared liposome (ch)-modified complexes included ACE2-siRNA/ch1, ACE2-shRNA/ch1, ACE2-(2ACE2-shRNA/ch1), (2ACE2-shRNA)-siRNA/ch1, (2ACE2-shRNA)-shRNA/ch1, and (2ACE2-shRNA)-(2ACE2-shRNA/ch1), abbreviated as ACE2-siRNA/ch1, ACE2-shRNA/ch1, 3ACE2-shRNA/ch1, 2ACE2-shRNA/siRNA/ch1, 2ACE2-2shRNA/ch1, and 4ACE2-2shRNA/ch1, respectively. As shown in FIG. 3, the ACE2 played a role in targeted delivery of siRNA/shRNA; the liposome and the PEG functioned as protection of siRNA/shRNA, slow release of siRNA/shRNA, and intracellular transfection of siRNA/shRNA, or an immunologic adjuvant.

### Example 2 Preparation of liposome-modified compound with liposome liposomal

When pH is greater than 8, the amino group of ACE2 reacted with pNP-PEG-DPPE (PEG-PE) to form a stable carbamate bond conjugate, which was then inserted into an outer membrane of the liposomes in a targeted and quantitative manner to obtain a liposome-modified compound. In this example, ACE2 fragments and siRNA were used to prepare a liposome-modified compound for ACE2-targeted delivery of siRNA (ACE2-siRNA).

### (1) Synthesis of ACE2

ACE2 or its fragments were synthesized using the ACE2 synthesis method described above.

### (2) Synthesis of pNP-PEG-DPPE

10 ml of a 20 mg/mL DPPE chloroform solution was placed in a 50 mL round-bottomed flask, and 0.65 mL of triethylamine (TEA) was added dropwise; about 4.0 g of a polyethylene glycol 3400 di-p-nitrophenyl carbonate [(pNP)₂-PEG₃₄₀₀] chloroform solution with a concentration of 200 mg/mL was added to an obtained mixture; the mixture was blown with nitrogen, sealed, protected from light, stirred magnetically overnight at a room temperature, the solvent was removed by evaporation under reduced pressure, and residual chloroform was removed in vacuum; 100 mL of a 0.01 mol/L HCl aqueous solution was added, and a transparent micellar solution was formed by an ultrasonic treatment. With the 0.01 mol/L HCl aqueous solution as an eluent, separation was conducted by CL-4B Sepharose to remove unreacted (pNP)₂-PEG₃₄₀₀ and released pNP; an eluate containing pNP-PEG-DPPE micelles was collected, lyophilized, and pNP-PEG-DPPE was characterized and quantified by TLC, HPLC, MS and NMR.

**(3) Synthesis of ACE2-PEG-DPPE:** 100 mg of the pNP-PEG-DPPE was dissolved in 10 mL of chloroform, placed in a 50 mL flask, and the chloroform was removed under reduced pressure on a rotary evaporator to form a lipid film, and the residual chloroform was removed by vacuum; 25 mg of ACE2 was dissolved in 4 mL of 0.01 mol/L HCl, a resulting mixture was added to a flask coated with the lipid film on the inner wall, incubated at a room temperature for 30 min, and shaken gently to disperse the lipid film. 20 mL of a 10 mum/L (pH 9.0) Tris buffer was added to a suspension, mixed well, and incubated at 4°C overnight under nitrogen protection. The samples were placed in a dialysis bag with a molecular mass of 5 kD, dialyzed in the 10 mmol/L (pH 7.4) Tris buffer for about 4 h, and then dialyzed in deionized water at 4°C for 24 h; the solution in the bag was lyophilized and stored in a -20°C refrigerator.

(4) **Synthesis of ACE2-siRNA/liposomal:** chloroform solutions of egg yolk phospholipid (ePC), cholesterol (Ch), distearoyl ethanolamine-polyethylene glycol 2000 (PEG₂₀₀₀-DSPE), and dioleoyl trimethylamine propane (DOTAP) were mixed in a molar ratio of 60:34:3.0:3.0; to label the lipid film, Rho-PE with a molar ratio of 0.1% to a total lipid mass was added in the above mixture, and chloroform was removed under reduced pressure to form the lipid film. A certain amount of the siRNA was dissolved in DEPC-treated ultrapure water, where an amount of the siRNA should ensure that the positive charge of DOTAP was completely neutralized. The phospholipid film was hydrated with an aqueous solution containing siRNA in a 50°C water bath for 30 min, to form an siRNA-encapsulated liposome. The preliminarily formed liposome was passed through 0.4 µm and 0.1 µm of polycarbonate nuclear pore membranes (Whatman) 10 times with a manual extrusion device (Avanti Polar Lipids), to prepare the liposomes with a uniform particle size. An appropriate amount of the ACE2-PEG-DPPE was dissolved in methanol, placed in a flask, and blow-dried with nitrogen to form a film; the prepared liposome suspension was added and treated in a water bath at 37°C for 2 h, such that the ACE2-PEG-DPPE was inserted into an outer membrane of the liposome and the ACE2 accounted for 0.5% to 1.0% of the total lipid (the molar ratio could be adjusted appropriately); a liposome (ch/lip) complex ACE2-shRNA/siRNA/lip as shown in FIG. 3 to FIG. 4 was prepared after dynamic laser scattering, cryo-etching electron microscopy, and nucleic acid electrophoresis.

The nCoVshRNA-2ACE2 was synthesized from the ACE2 or its polypeptides with the shRNA/siRNA, and then further modified by the liposomes.

### VII. Validation of the compound (nCoVshRNA·2ACE2)

### 1. In vitro verification of broad-spectrum antiviral effect

### (1) Preparation of virus solution

The virus strains were added to a DMEM medium (10% FBS) of Vero E6 cells grown to 30% confluence, and incubated in a 36°C, 5% CO₂ incubator for 5 d to 7 d; when an cytopathic effect (CPE) occurred, the virus was isolated and then prepared by a medium into a 10³ TCID₅₀/ml to 10⁵ TCID₅₀/ml virus solution for later use. According to this, virus solutions of two variant strains B.1.617.1 and B.1.617.2 of the COVID-19 virus were prepared separately to verify whether the nCoVshRNA-2ACE2 was effective against two or more variant viruses containing a same conserved gene, so as to prove whether the shRNA/siRNA of the present disclosure had a broad-spectrum antiviral effect by targeting the conserved gene.

### (2) Co-culture of compound (nCoVshRNA·2ACE2) with virus

An experimental group and a control group were set up to test an effect of the compounds against the B.1.617.1 and B.1.617.2. Each group was inoculated with a 8-well plate, and 2×10⁵ Vero-E6 cells and 2 mL of a DMEM medium (10% FBS) were added to each well, and then incubated in a 36°C and 5% CO₂ incubator to 30% confluence, followed by changing the medium; meanwhile, the tested compound and the virus solutions of B.1.617.1, and B.1.617.2 strains were added.

The experimental groups included: a 2ACE2-shRNA3 group (0.1 nmol 2ACE2-shRNA3 + 0.6 ml virus solution), a 2ACE2-shRNA3/ch1 group (0.1 nmol 2ACE2-shRNA3/ch1 + 0.6 ml virus solution), a 4ACE2-2shRNA3/ch1 group (0.1 nmol 4ACE2-2shRNA3/ch1 + 0.6 ml virus solution), an ACE2-siRNA3/lip group (0.1 nmol ACE2-siRNA3/lip + 0.6 ml virus solution); the control group included: a naked shRNA3 group (0.1 nmol naked shRNA3 + 0.6 ml virus solution), a naked siRNA3 group (0.1 nmol naked siRNA3 + 0.6 ml virus solution), an ACE2 control group (0.1 nmol ACE2 + 0.6 ml virus solution), a positive control group (0.6 ml virus solution), and a negative control group (0.6 ml DMEM medium) (Tables 1 to 6). Similarly, experiments with 2ACE2-shRNA5 and 2ACE2-shRNA8 were conducted (Tables 1a to 6a).

After 1 h, 24 h, and 72 h of incubation, a supernatant was collected from each group, and then diluted at 1:4, 1:12, 1:36, 1:108, 1:324, 1:972, 1:2916, and 1:8748 to conduct RT-PCR detection.

### (3) Real-time fluorescent RT-PCR detection of viral RNA in each group

Viral nucleic acid extraction and nucleic acid (ORF1ab/N) detection were conducted according to kit instructions.

### (4) Detection results of viral RNA

### 1) Test results of strain B.1.617.1

As shown in Table 1, after culturing the cells in each group for 1 h, a viral RNA titer of the negative control group was negative, a viral RNA titer of the positive control group was 1:36, and viral RNA titers of the other groups each were 1:12.

As shown in Table 2, after culturing the cells in each group for 24 h, a viral RNA titer of the negative control group was still negative, and a viral RNA titer of each control group was 1:324 to 1:2916; while viral RNA titers of the experimental groups each were 1:108, which were significantly lower than that of the positive control group and the ACE2 control group (p<0.01).

As shown in Table 3, after culturing the cells in each group for 72 h, a viral RNA titer in the negative control group was still negative, a viral RNA titer of each control group was greater than 1:2916, and a viral RNA titer of the control group was greater than 1:2916 to 1:8748; while a viral RNA titer of the experimental group was 1:324 to 1:972, which was significantly lower than that of the control group (p<0.01).

Tables 1 to 3 showed that the experimental group had an obvious anti-B.1.617.1 effect, indicating that shRNA or siRNA ligated to ACE2 could be delivered to target cells for RNA interference. However, the shRNA or siRNA that was not ligated to ACE2 could not enter the target cells, and could not play a role of RNA interference extracellularly. The results in Tables 1a to 3a were basically consistent with those in Tables 1 to 3.

**Table 1 Virus RNA RT-PCR detection results in medium after compound (shRNA3) co-cultured with strain B.1.617.1 for 1 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA3 | + | + | - | - | - | - | - | - |
| 2ACE2-shRNA3/ch1 | + | + | - | - | - | - | - | - |
| 4ACE2-2shRNA3/ch1 | + | + | - | - | - | - | - | - |
| ACE2-siRNA3/lip | + | + | - | - | - | - | - | - |
| naked shRNA3 | + | + | - | - | - | - | - | - |
| naked siRNA3 | + | + | - | - | - | - | - | - |
| ACE2 control | + | + | - | - | - | - | - | - |
| Positive control | + | + | + | - | - | - | - | - |
| Negative control | - | - | - | - | - | - | - | - |

**Table 1a Virus RNA RT-PCR detection results in medium after 2ACE2-shRNA5/8 co-cultured with strain B.1.617.1 for 1 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA5 | + | - | - | - | - | - | - | - |
| 2ACE2-shRNA5/ch1 | + | - | - | - | - | - | - | - |
| 2ACE2-shRNA8 | + | - | - | - | - | - | - | - |
| 2ACE2-shRNA8/ch1 | + | - | - | - | - | - | - | - |
| naked shRNA5 | + | - | - | - | - | - | - | - |
| naked shRNA8 | + | - | - | - | - | - | - | - |
| ACE2 control | + | - | - | - | - | - | - | - |
| Positive control | + | + | - | - | - | - | - | - |
| Negative control | - | - | - | - | - | - | - | - |

**Table 2 Virus RNA RT-PCR detection results in medium after compound (shRNA3) co-cultured with strain B.1.617.1 for 24 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA3 | + | + | + | + | - | - | - | - |
| 2ACE2-shRNA3/ch1 | + | + | + | + | - | - | - | - |
| 4ACE2-2shRNA3/ch1 | + | + | + | + | - | - | - | - |
| ACE2-siRNA3/lip | + | + | + | + | - | - | - | - |
| naked shRNA3 | + | + | + | + | + | - | - | - |
| naked siRNA3 | + | + | + | + | + | - | - | - |
| ACE2 control | + | + | + | + | + | + | + | - |
| Positive control | + | + | + | + | + | + | - | - |
| Negative control | - | - | - | - | - | - | - | - |

**Table 2a Virus RNA RT-PCR detection results in medium after 2ACE2-shRNA5/8 co-cultured with strain B.1.617.1 for 24 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA5 | + | + | + | - | - | - | - | - |
| 2ACE2-shRNA5/ch1 | + | + | + | - | - | - | - | - |
| 2ACE2-shRNA8 | + | + | + | - | - | - | - | - |
| 2ACE2-shRNA8/ch1 | + | + | + | - | - | - | - | - |
| naked shRNA5 | + | + | + | + | - | - | - | - |
| naked shRNA8 | + | + | + | + | - | - | - | - |
| ACE2 control | + | + | + | + | + | + | - | - |
| Positive control | + | + | + | + | + | - | - | - |
| Negative control | - | - | - | - | - | - | - | - |

**Table 3 Virus RNA RT-PCR detection results in medium after compound (shRNA3) co-cultured with strain B.1.617.1 for 72 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA3 | + | + | + | + | + | + | - | - |
| 2ACE2-shRNA3/ch1 | + | + | + | + | + | - | - | - |
| 4ACE2-2shRNA3/ch1 | + | + | + | + | + | + | - | - |
| ACE2-siRNA3/lip | + | + | + | + | + | - | - | - |
| naked shRNA3 | + | + | + | + | + | + | + | - |
| naked siRNA3 | + | + | + | + | + | + | + | + |
| ACE2 control | + | + | + | + | + | + | + | + |
| Positive control | + | + | + | + | + | + | + | + |
| Negative control | - | - | - | - | - | - | - | - |

**Table 3a Virus RNA RT-PCR detection results in medium after 2ACE2-shRNA5/8 co-cultured with strain B.1.617.1 for 72 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA5 | + | + | + | + | - | - | - | - |
| 2ACE2-shRNA5/ch1 | + | + | + | + | - | - | - | - |
| 2ACE2-shRNA8 | + | + | + | + | + | - | - | - |
| 2ACE2-shRNA8/ch1 | + | + | + | + | - | - | - | - |
| naked shRNA5 | + | + | + | + | + | + | + | - |
| naked shRNA8 | + | + | + | + | + | + | + | - |
| ACE2 control | + | + | + | + | + | + | + | + |
| Positive control | + | + | + | + | + | + | + | + |
| Negative control | - | - | - | - | - | - | - | - |

### 2) Test results of strain B.1.617.2

As shown in Table 4, after culturing the cells in each group for 1 h, a viral RNA titer of the negative control group was negative, viral RNA titers of the positive control group and the ACE2 control group each were 1:36, and viral RNA titers of the other groups each were 1:12. As shown in Table 5, after culturing the cells in each group for 24 h, a viral RNA titer of the negative control group was still negative, and a viral RNA titer of each control group was 1:324 to 1:972; while viral RNA titers of the 4 experimental groups were 1:36 to 1:108, which were significantly lower than that in the control group (p<0.01). As shown in Table 6, after culturing the cells in each group for 72 h, a viral RNA titer of the negative control group was still negative, and a viral RNA titer of each control group was 1:2916 to 1:8748; while viral RNA titers of the 4 experimental groups were 1:324 in 2 groups and 1:972 in the other 2 groups, which were still significantly different from that in the control group (p<0.01). Tables 4 to 6 showed that the experimental group had an obvious anti-B.1.617.2 effect, indicating that shRNA or siRNA ligated to ACE2 could be delivered to target cells for RNA interference. However, the shRNA or siRNA that was not ligated to RBD could not enter the target cells, and could not play a role of RNA interference. The results in Tables 4a to 6a were basically consistent with those in Tables 4 to 6. Tables 1 to 6 and 1a to 6a showed that the experimental group (nCoVshRNA·2ACE2) had anti-B.1.617.1 and anti-B.1.617.2 effects, indicating that the nCoVshRNA·2ACE2 targeting conserved genes in the experimental group had a broad-spectrum anti-variant strain effect.

**Table 4 Virus RNA RT-PCR detection results in medium after compound (shRNA3) co-cultured with strain B.1.617.2 for 1 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA3 | + | + | - | - | - | - | - | - |
| 2ACE2-shRNA3/ch1 | + | + | - | - | - | - | - | - |
| 4ACE2-2shRNA3/ch1 | + | + | - | - | - | - | - | - |
| ACE2-siRNA3/lip | + | + | - | - | - | - | - | - |
| naked shRNA3 | + | + | - | - | - | - | - | - |
| naked siRNA3 | + | + | - | - | - | - | - | - |
| ACE2 control | + | + | + | - | - | - | - | - |
| Positive control | + | + | + | - | - | - | - | - |
| Negative control | - | - | - | - | - | - | - | - |

**Table 4a Virus RNA RT-PCR detection results in medium after 2ACE2-shRNA5/8 co-cultured with strain B.1.617.2 for 1 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA5 | + | - | - | - | - | - | - | - |
| 2ACE2-shRNA5/ch1 | + | - | - | - | - | - | - | - |
| 2ACE2-shRNA8 | + | - | - | - | - | - | - | - |
| 2ACE2-shRNA8/ch1 | + | - | - | - | - | - | - | - |
| naked shRNA5 | + | - | - | - | - | - | - | - |
| naked shRNA8 | + | - | - | - | - | - | - | - |
| ACE2 control | + | + | - | - | - | - | - | - |
| Positive control | + | + | - | - | - | - | - | - |
| Negative control | - | - | - | - | - | - | - | - |

**Table 5 Virus RNA RT-PCR detection results in medium after compound (shRNA3) co-cultured with strain B.1.617.2 for 24 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA3 | + | + | + | + | - | - | - | - |
| 2ACE2-shRNA3/ch1 | + | + | + | - | - | - | - | - |
| 4ACE2-2shRNA3/ch1 | + | + | + | + | - | - | - | - |
| ACE2-siRNA3/lip | + | + | + | - | - | - | - | - |
| naked shRNA3 | + | + | + | + | + | - | - | - |
| naked siRNA3 | + | + | + | + | + | - | - | - |
| ACE2 control | + | + | + | + | + | + | - | - |
| Positive control | + | + | + | + | + | - | - | - |
| Negative control | - | - | - | - | - | - | - | - |

**Table 5a Virus RNA RT-PCR detection results in medium after 2ACE2-shRNA5/8 co-cultured with strain B.1.617.2 for 24 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA5 | + | + | + | - | - | - | - | - |
| 2ACE2-shRNA5/ch1 | + | + | - | - | - | - | - | - |
| 2ACE2-shRNA8 | + | + | + | - | - | - | - | - |
| 2ACE2-shRNA8/ch1 | + | + | + | - | - | - | - | - |
| naked shRNA5 | + | + | + | + | - | - | - | - |
| naked shRNA8 | + | + | + | + | - | - | - | - |
| ACE2 control | + | + | + | + | + | + | + | - |
| Positive control | + | + | + | + | + | + | - | - |
| Negative control | - | - | - | - | - | - | - | - |

**Table 6 Virus RNA RT-PCR detection results in medium after compound (shRNA3) co-cultured with strain B.1.617.2 for 72 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA3 | + | + | + | + | + | + | - | - |
| 2ACE2-shRNA3/ch1 | + | + | + | + | + | - | - | - |
| 4ACE2-2shRNA3/ch1 | + | + | + | + | + | + | - | - |
| ACE2-siRNA3/lip | + | + | + | + | + | - | - | - |
| naked shRNA3 | + | + | + | + | + | + | + | - |
| naked siRNA3 | + | + | + | + | + | + | + | - |
| ACE2 control | + | + | + | + | + | + | + | + |
| Positive control | + | + | + | + | + | + | + | - |
| Negative control | - | - | - | - | - | - | - | - |

**Table 6a Virus RNA RT-PCR detection results in medium after 2ACE2-shRNA5/8 co-cultured with strain B.1.617.2 for 72 h**

| Group | Viral RNA detection results of different dilutions in medium (+/-) | | | | | | | |
|---|---|---|---|---|---|---|---|---|
| | 1:4 | 1:12 | 1:36 | 1:108 | 1:324 | 1:972 | 1:2916 | 1:8748 |
| 2ACE2-shRNA5 | + | + | + | + | + | - | - | - |
| 2ACE2-shRNA5/ch1 | + | + | + | + | - | - | - | - |
| 2ACE2-shRNA8 | + | + | + | + | + | - | - | - |
| 2ACE2-shRNA8/ch1 | + | + | + | + | - | - | - | - |
| naked shRNA5 | + | + | + | + | + | + | + | - |
| naked shRNA8 | + | + | + | + | + | + | + | - |
| ACE2 control | + | + | + | + | + | + | + | + |
| Positive control | + | + | + | + | + | + | + | + |
| Negative control | - | - | - | - | - | - | - | - |

### 2. In vivo verification of targeted delivery function of ACE2

### (1) Animal grouping and inoculation

**Animal grouping:** female BALB/c mice in an SPF grade of 6 to 8 weeks and about 40 g were selected; taking the 2ACE2-shRNA3 as an example, the mice were randomly divided into a 2ACE2-shRNA3 (shRNA5/8) group (inoculated with 2ACE2-shRNA3 + B.1.617.2), a 2ACE2-shRNA3/chl group (inoculated with 2ACE2-shRNA3/chl + B.1.617.2), a 4ACE2-2shRNA3/chl group (inoculated with 4ACE2-2shRNA3/chl + B.1.617.2), an ACE2-siRNA3/lip group (inoculated with ACE2-siRNA3/lip + B.1.617.2), a shRNA3 group (inoculated with shRNA3 + B.1.617.2), a positive control group (inoculated with B.1.617.2 + normal saline), and a negative control group (inoculated with normal saline only), with 20 mice in each group.

**Animal inoculation:** the mice each were inoculated with 40 µl of a B.1.617.2 strain virus solution with a titer of 10⁵/ml TCID₅₀ by nasal spray, while the negative control group was inoculated with 40 µl of a normal saline by nasal spray. The mice were anesthetized by intraperitoneal injection of a 5% chloral hydrate solution, and 0.1 nmol of the 2ACE2-shRNA, 2ACE2-shRNA/ch1, 4ACE2-2shRNA/ch1, ACE2-siRNA/lip, and shRNA were slowly injected into the trachea of the mice separately. On the 7th day after infection, 10 mice in each group were sacrificed for virus detection; the remaining 10 mice in each group were used to observe antibodies.

### (2) Detection of virus at percentage of median tissue culture infective dose (TCID₅₀) of cells

A 10% homogenate was prepared from a lung tissue of the mice sacrificed on the 7th day after infection, and 100 pl of the homogenate was centrifuged to remove a supernatant, the homogenate was diluted 10-fold successively, and inoculated in a 96-well plate with VeroE6 growing in a single layer at 30 µl per well and 4 wells per dilution; the homogenate was gently shaken, adsorbed at 37°C for 1 h, washed with a Hank's solution, added with a medium, and then incubated in a 37°C CO₂ incubator; a cytopathic effect (CPE) was observed, and a percentage of VeroE6 TCID₅₀ in each group was calculated, where a higher percentage meant a higher virus content (Tables 7 to 13).

**Table 7 Percentage of TCID₅₀ by VeroE6 in mice lung tissue homogenate in 2ACE2-shRNA3 group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 26 | 14 | 14/40 | 35.0 |
| 10² | 40 | 31 | 9 | 9/40 | 22.5 |
| 10³ | 40 | 33 | 7 | 7/40 | 17.5 |
| 10⁴ | 40 | 37 | 3 | 3/40 | 7.5 |

**Table 8 Percentage of TCID₅₀ by VeroE6 in mice lung tissue homogenate in 2ACE2-shRNA3/ch1 group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 29 | 11 | 11/40 | 27.5 |
| 10² | 40 | 31 | 9 | 9/40 | 22.5 |
| 10³ | 40 | 34 | 6 | 6/40 | 15.0 |
| 10⁴ | 40 | 38 | 2 | 2/40 | 5.0 |

**Table 9 Percentage of TCID₅₀ by VeroE6 in mice lung tissue homogenate in 4ACE2-2shRNA3/ch1 group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 27 | 13 | 13/40 | 32.5 |
| 10² | 40 | 31 | 9 | 9/40 | 22.5 |
| 10³ | 40 | 35 | 5 | 5/40 | 12.5 |
| 10⁴ | 40 | 37 | 3 | 3/40 | 7.5 |

**Table 10 Percentage of TCID₅₀ by VeroE6 in mice lung tissue homogenate in ACE2-siRNA3/lip group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 28 | 12 | 12/40 | 30.0 |
| 10² | 40 | 31 | 9 | 9/40 | 22.5 |
| 10³ | 40 | 34 | 6 | 6/40 | 15.0 |
| 10⁴ | 40 | 36 | 4 | 4/40 | 10.0 |

**Table 11 Percentage of TCID₅₀ by VeroE6 in mice lung tissue homogenate in shRNA3 group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 2 | 38 | 38/40 | 95.0 |
| 10² | 40 | 4 | 36 | 36/40 | 90.0 |
| 10³ | 40 | 9 | 31 | 31/40 | 77.5 |
| 10⁴ | 40 | 13 | 27 | 27/40 | 67.5 |

**Table 12 Percentage of TCID₅₀ by VeroE6 in mice lung tissue homogenate in positive control group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 2 | 38 | 38/40 | 95.0 |
| 10² | 40 | 3 | 37 | 37/40 | 92.5 |
| 10³ | 40 | 7 | 33 | 33/40 | 82.5 |
| 10⁴ | 40 | 11 | 29 | 29/40 | 72.5 |

**Table 13 Percentage of TCID₅₀ by VeroE6 in mice lung tissue homogenate in negative control group**

| Tissue homogenate dilution | Number of inoculated cells (4 wells×10 cases) | Total observed results | | Infection ratio | Infection rate |
|---|---|---|---|---|---|
| | | Normal well | Lesion well | | |
| 10¹ | 40 | 38 | 2 | 2/40 | 5.0 |
| 10² | 40 | 38 | 2 | 2/40 | 5.0 |
| 10³ | 40 | 39 | 1 | 1/40 | 2.5 |
| 10⁴ | 40 | 39 | 1 | 1/40 | 2.5 |

### (3) Effect of ACE2-targeted delivery

As was seen from Tables 7 to 13, percentages of VeroE6 TCID₅₀ (10¹ in each well) induced by lung homogenate in each group were as follows: 2ACE2-shRNA3 group was 35.0%, 2ACE2-shRNA3/ch1 group was 27.5%, 4ACE2-2shRNA3/ch1 group was 32.5%, ACE2-siRNA3/lip group was 30.0%, shRNA3 group was 95.0%, positive control group was 95.0%, and negative control group was 5.0%. Since RNAi mainly occurred in the cytoplasm, shRNA3 in the shRNA3 group was not easy to pass through the cell membrane, so as to have a little effect on the RNAi, and results were consistent with the positive control group; meanwhile, the shRNA3 in the 2ACE2-shRNA3 group, 2ACE2-shRNA3/ch1 group, 4ACE2-2shRNA3/ch1 group, and ACE2-siRNA3/lip group had a better RNAi effect due to targeted delivery of the ACE2 to the target cytoplasm, and the percentage of VeroE6 TCID₅₀ was significantly different from that of the positive control group (p<0.05).

A TCID₅₀ assay was conducted separately on the 2ACE2-shRNA8 and 2ACE2-shRNA8/ch1 according to the TCID₅₀ assay method above. The results showed that the 2ACE2-shRNA8 group, 2ACE2-shRNA8/ch1 group, shRNA8 group, positive control group, and negative control group had TCID₅₀ of 25%, 17.5%, 82.5%, 95%, and 7.5%, respectively, and the TCID₅₀ of experimental groups was significantly lower than that of the positive control group.

### 3. Detection and functional verification of ACE2-Ab

### (1) Sample and detection method

The venous blood of the remaining 10 mice in each group was collected at the 2nd, 4th, and 6th weeks, a serum was separated, and sera of a same week in each group were mixed, and then stored at -20°C for future use. The ACE2-Ab was determined by a double-antigen sandwich method according to instructions of the kit.

### (2) Detection results

It was seen from Table 14 that the ACE2-Ab in the lung tissue homogenate of mice in each experimental group (with ACE2) was significantly higher than that in the control group (without ACE2) (p<0.05), indicating that the ACE2 of experimental groups could stimulate the mice to produce ACE2-Ab, and a content of the ACE2-Ab was related to ACE2 molecular composition and liposome modification.

**Table 14 ACE2-Ab detection results (ng/L) of mouse lung tissue homogenate in each experimental group (with ACE2)**

| Group | ACE2 molecule | Weeks and detection results | | | |
|---|---|---|---|---|---|
| | | 2nd week | 4th week | 6th week | P value |
| 2ACE2-shRNA3 | 2 | 45.11+16.27* | 47.13±14.12* | 48.67±15.46* | *p<0.05 |
| 2ACE2-shRNA3/ch1 | 2 | 55.18±16.77* | 69.17±17.35* | 73.78±17.13* | *p<0.05 |
| 4ACE2-2shRNA/ch1 | 4 | 62.12±15.17* | 72.15±17.33* | 73.66±16.27* | *p<0.05 |
| ACE2-siRNA3/lip | 1 | 41.22±14.82 | 44.57±16.19 | 46.16±15.23 | *p<0.05 |
| shRNA3 | None | 11.23±5.76 | 12.615±6.32 | 11.78±5.75 | |

### (3) Functional verification of ACE2-Ab

The 2ACE2-shRNA group, 2ACE2-shRNA/ch1 group, 4ACE2-2shRNA/ch1 group, and ACE2-siRNA/lip group were used as experimental groups (all containing ACE2-Ab), while the shRNA group was used as a control group (containing virus but not ACE2-Ab). The sera of 2nd, 4th, and 6th week in each group after ACE2-Ab detection were mixed, a mixed serum in each group were double-diluted, and 30 µl of each diluted serum was inoculated in a 96-well plate with VeroE6 growing in a single layer; each experimental group was simultaneously inoculated with 30 µl of an undiluted mixed serum of the shRNA group, while the negative control group was inoculated with 30 µl of a normal saline only. The samples in each group were shaken and mixed well, adsorbed at 37°C for 1 h, washed with the Hank's solution, added with a medium, and incubated in a 37°C CO₂ incubator; a cytopathic effect (CPE) was observed within 1 week, where "+" indicated the normal cell growth, as shown in Table 15.

**Table 15 Results of antiviral infection caused by ACE2-Ab neutralization of ACE2 on surface of VeroE6 cells (+/CPE)**

| Group | Dilution of serum (ACE2-Ab) inhibiting virus-induced VeroE6 CPE | | | | | | | | |
|---|---|---|---|---|---|---|---|---|---|
| | 1:2 | 1:4 | 1:8 | 1:16 | 1:32 | 1:64 | 1:128 | 1:256 | 1:512 |
| 2ACE2-shRNA3 | + | + | + | + | + | CPE | CPE | CPE | CPE |
| 2ACE2-shRNA3/ch1 | + | + | + | + | + | CPE | CPE | CPE | CPE |
| 4ACE2-2shRNA3/ch1 | + | + | + | + | + | + | CPE | CPE | CPE |
| ACE2-siRNA3/lip | + | + | + | + | + | CPE | CPE | CPE | CPE |
| shRNA3 | CPE | CPE | CPE | CPE | CPE | CPE | + | + | + |
| Positive control | CPE | CPE | CPE | CPE | CPE | CPE | CPE | CPE | CPE |
| Negative control | + | + | + | + | + | + | + | + | + |

As was seen from Table 15, since the negative control group was not inoculated with virus, there was no CPE in each well of VeroE6; the positive control group was inoculated with the virus without ACE2-Ab neutralization (the mixed serum in shRNA3 group was not diluted), such that VeroE6 in each well produced CPE; since the shRNA3 group had no neutralization effect of ACE2-Ab, VeroE6 did not have CPE only after a virus-containing self-serum was diluted at 1:128; though the experimental groups were also inoculated with the virus like the positive control group, but due to the neutralization effect of ACE2-Ab, the VeroE6 showed CPE only when the serum (ACE2-Ab content) was diluted at not less than 1:64. It showed that the ACE2-Ab could neutralize the viral receptor ACE2 on the surface of VeroE6 cells, thereby producing an antiviral effect.

In the present disclosure, shRNA plays a role in anti-variant strain effects; ACE2 plays a role in targeted delivery of shRNA, bivalent binding to RBD, competitive inhibition of virus infection through RBD, protection of shRNA3, and stimulation of host to produce ACE2-Ab that can block ACE2. Moreover, multi-molecular ACE2 and adjuvant shRNA3 and liposomes each can promote the production and effect of ACE2-Ab.

## Claims

1. An *in vitro* synthesis method of a targeted drug nCoVshRNA-2ACE2, comprising the following steps: synthesizing a shRNA with a sense strand and an antisense strand of an RNAi sequence, extending a terminus of the sense strand and/or the antisense strand constituting the shRNA, and ligating to a ACE2 polypeptide to synthesize a drug that delivers the shRNA by the ACE2 polypeptide, wherein the RNAi sequence is a consensus conserved gene of a COVID-19 virus and a variant strain thereof, such that the shRNA synthesized with the sense strand and the antisense strand of the RNAi sequence is targeted to interfere with the conserved gene, resulting in a broadspectrum RNAi effect.

2. The synthesis method of a targeted drug nCoVshRNA-2ACE2 according to claim 1, wherein a process of synthesizing the shRNA specifically comprises: synthesizing two complementary sense strand and antisense strand of 21 nt to 25 nt, and synthesize a base sequence that act as a spacer; ligating the sense strand and antisense strand and the base sequence to form a small hairpin-shaped shRNA with a loop that is in a middle position and separated by the base sequence; and ligating the ACE2 polypeptide to each single strand of the shRNA.

3. The synthesis method of a targeted drug nCoVshRNA-2ACE2 according to claim 1 or 2, for targeted delivery of the shRNA serving as a consensus RNAi target of a coronavirus; further comprising ligating the ACE2 with antigenic properties into a dimer ACE2 by the shRNA with immunologic adjuvant properties.

4. The synthesis method of a targeted drug nCoVshRNA-2ACE2 according to claim 1 or 3, wherein the sense strand and antisense strand for synthesizing the shRNA comprises RNAi sequences SEQ ID NO: 7 to SEQ ID NO: 58 of the COVID-19 virus; and the sense strand and antisense strand comprises preferred consensus sequences SEQ ID NO: 8 to SEQ ID NO: 10, SEQ ID NO: 16 to SEQ ID NO: 18, SEQ ID NO: 20 to SEQ ID NO: 22, SEQ ID NO: 30 to SEQ ID NO: 32, and SEQ ID NO: 41 to SEQ ID NO: 58 of the COVID-19 virus.

5. The synthesis method of a targeted drug nCoVshRNA-2ACE2 according to claim 1 or 4, wherein the sense strand and antisense strand for synthesizing the shRNA is preferably SEQ ID NO: 16 to SEQ ID NO: 18 and SEQ ID NO: 49 to SEQ ID NO: 51 targeting an *N* gene of the COVID-19 virus, SEQ ID NO: 20 to SEQ ID NO: 22 and SEQ ID NO: 52 to SEQ ID NO: 54 targeting an *ORFIab* gene of the COVID-19 virus, and SEQ ID NO: 30 to SEQ ID NO: 32 and SEQ ID NO: 56 to SEQ ID NO: 58 targeting an S gene of the COVID-19 virus; the sense strand and antisense strand for synthesizing the shRNA is more preferably SEQ ID NO: 16 and SEQ ID NO: 49 targeting the N gene, SEQ ID NO: 21 and SEQ ID NO: 52 targeting the *ORFIab* gene, and SEQ ID NO: 30 targeting the S gene; and the sense strand and antisense strand for synthesizing the shRNA is further more preferably SEQ ID NO: 49, and SEQ ID NO: 30.

6. The synthesis method of a targeted drug nCoVshRNA-2ACE2 according to claim 1 or 5, wherein a process of synthesizing the nCoVshRNA-2ACE2 specifically comprises: ligating the ACE2 polypeptide with the shRNA and/or the sense strand and antisense strand to form a compound, or inserting the shRNA and/or the sense strand and antisense strand into the ACE2 or the polypeptide thereof and ligating to form the compound; and modifying the compound with a liposome or lipid nanoparticles.

7. The synthesis method of a targeted drug nCoVshRNA-2ACE2 according to claim 1 or 6, wherein the ACE2 comprises extracellular ACE2 at amino acids 1 to 740, transmembrane ACE2 at amino acids 741 to 763, intracellular ACE2 at amino acids 764 to 805, or an amino acid sequence-optimized ACE2 polypeptide.

8. The synthesis method of a targeted drug nCoVshRNA-2ACE2 according to claim 1 or 6, wherein the ligating or the synthesizing comprises ligating the ACE2 with a 3'-end of the antisense strand and a 5'-end or a 3'-end of the sense strand of the shRNA; the ligating or the synthesizing comprises conducting the ligating by chemical coupling or covalent coupling with a carboxyhydrazone bond, a disulfide bond, a phosphodiester bond, a phosphorodithioate bond, a thioether bond, an oxime bond, an amide bond, and a maleimide-thiol bond that have a spacer arm; the ligating or the synthesizing comprises ligating the ACE2 polypeptide separately on the sense strand and the antisense strand of the shRNA.

9. The synthesis method of a targeted drug nCoVshRNA-2ACE2 according to claim 1 or 8, wherein the nCoVshRNA-2ACE2 comprises the sense strand and antisense strand/shRNA prepared with SEQ ID NO: 7 to SEQ ID NO: 58, or a ligation product ACE2-shRNA with the ACE2, or a liposome complex of the ACE2-shRNA; ACE2 shRNA comprises 2ACE2-shRNA5 having SEQ ID NO:49; 2ACE2-shRNA8 having SEQ ID NO:30, , the liposome complex of the ACE2-shRNA comprises 2ACE2-shRNA5/cholesterol, 2ACE2-shRNA8/cholesterol, and/or ACE2-shRNA liposome.

## Patentansprüche

1. In-vitro-Syntheseverfahren eines zielgerichteten Arzneimittels nCoVshRNA-2ACE2, umfassend die folgenden Schritte: Synthetisieren einer shRNA mit einem Sense-Strang und einem Antisense-Strang einer RNAi-Sequenz, Verlängern eines Endes des Sense-Strangs und/oder des Antisense-Strangs, die die shRNA bilden, und Ligieren an ein ACE2-Polypeptid zur Synthese eines Arzneimittels, das die shRNA durch das ACE2-Polypeptid abgibt, wobei die RNAi-Sequenz ein gemeinsames konserviertes Gen eines COVID-19-Virus und einer Stammvariante davon ist, so dass die mit dem Sense-Strang und dem Antisense-Strang der RNAi-Sequenz synthetisierte shRNA darauf ausgerichtet ist, das konservierte Gen zu stören, was zu einem Breitspektrum-RNAi-Effekt führt.

2. Syntheseverfahren eines zielgerichteten Arzneimittels nCoVshRNA-2ACE2 nach Anspruch 1, wobei ein Prozess zur Synthese der shRNA insbesondere Folgendes umfasst: Synthetisieren von zwei komplementären Sense- und Antisense-Strängen mit einer Länge von 21 nt bis 25 nt und Synthetisieren einer Basensequenz, die als Spacer fungiert; Ligieren des Sense-Strangs und des Antisense-Strangs und der Basensequenz, um eine kleine haarnadelförmige shRNA mit einer in mittlerer Position befindlichen Schleife zu bilden, die durch die Basensequenz getrennt ist; und Ligieren des ACE2-Polypeptids an jeden einzelnen Strang der shRNA.

3. Syntheseverfahren eines zielgerichteten Arzneimittels nCoVshRNA-2ACE2 nach Anspruch 1 oder 2 zur zielgerichteten Abgabe der shRNA, die als gemeinsames RNAi-Target eines Coronavirus dient; ferner umfassend Ligieren des ACE2 mit antigenen Eigenschaften zu einem Dimer ACE2 durch die shRNA mit immunologischen Adjuvans-Eigenschaften.

4. Syntheseverfahren eines zielgerichteten Arzneimittels nCoVshRNA-2ACE2 nach Anspruch 1 oder 3, wobei der Sense-Strang und der Antisense-Strang zur Synthese der shRNA die RNAi-Sequenzen SEQ ID NO: 7 bis SEQ ID NO: 58 des COVID-19-Virus umfassen; und der Sense-Strang und Antisense-Strang bevorzugte gemeinsame Sequenzen SEQ ID NO: 8 bis SEQ ID NO: 10, SEQ ID NO: 16 bis SEQ ID NO: 18, SEQ ID NO: 20 bis SEQ ID NO: 22, SEQ ID NO: 30 bis SEQ ID NO: 32 und SEQ ID NO: 41 bis SEQ ID NO: 58 des COVID-19-Virus umfassen.

5. Syntheseverfahren eines zielgerichteten Arzneimittels nCoVshRNA-2ACE2 nach Anspruch 1 oder 4, wobei der Sense-Strang und der Antisense-Strang zur Synthese der shRNA vorzugsweise SEQ ID NO: 16 bis SEQ ID NO: 18 und SEQ ID NO: 49 bis SEQ ID NO: 51, die auf ein N-Gen des COVID-19-Virus abzielen, SEQ ID NO: 20 bis SEQ ID NO: 22 und SEQ ID NO: 52 bis SEQ ID NO: 54, die auf ein *ORFlab*-Gen des COVID-19-Virus abzielen, und SEQ ID NO: 30 bis SEQ ID NO: 32 und SEQ ID NO: 56 bis SEQ ID NO: 58, die auf ein S-Gen des COVID-19-Virus abzielen, sind; der Sense-Strang und der Antisense-Strang zur Synthese der shRNA vorzugsweise SEQ ID NO: 16 und SEQ ID NO: 49, die auf das N-Gen abzielen, SEQ ID NO: 21 und SEQ ID NO: 52, die auf das *ORFlab*-Gen abzielen, und SEQ ID NO: 30, die auf das S-Gen abzielt, sind; und der Sense-Strang und der Antisense-Strang zur Synthese der shRNA weiter bevorzugter SEQ ID NO: 49 und SEQ ID NO: 30 sind.

6. Syntheseverfahren eines zielgerichteten Arzneimittels nCoVshRNA-2ACE2 nach Anspruch 1 oder 5, wobei ein Prozess zur Synthese des nCoVshRNA-2ACE2 insbesondere Folgendes umfasst: Ligieren des ACE2-Polypeptids mit der shRNA und/oder dem Sense-Strang und Antisense-Strang zum Bilden einer Verbindung, oder Einfügen der shRNA und/oder des Sense-Strangs und Antisense-Strangs in das ACE2 oder das Polypeptid davon und Ligieren zum Bilden der Verbindung; und Modifizieren der Verbindung mit einem Liposom oder Lipid-Nanopartikeln.

7. Syntheseverfahren eines zielgerichteten Arzneimittels nCoVshRNA-2ACE2 nach Anspruch 1 oder 6, wobei das ACE2 extrazelluläres ACE2 an den Aminosäuren 1 bis 740, transmembranes ACE2 an den Aminosäuren 741 bis 763, intrazelluläres ACE2 an den Aminosäuren 764 bis 805 oder ein Aminosäuresequenz-optimiertes ACE2-Polypeptid umfasst.

8. Syntheseverfahren eines zielgerichteten Arzneimittels nCoVshRNA-2ACE2 nach Anspruch 1 oder 6, wobei das Ligieren oder das Synthetisieren das Ligieren des ACE2 mit einem 3'-Ende des Antisense-Strangs und einem 5'-Ende bzw. einem 3'-Ende des Sense-Strangs der shRNA umfasst; das Ligieren oder das Synthetisieren das Durchführen der Ligierung durch chemische Kopplung oder kovalente Kopplung mit einer Carboxyhydrazon-Bindung, einer Disulfid-Bindung, einer Phosphodiester-Bindung, einer Phosphordithioat-Bindung, einer Thioether-Bindung, einer Oxim-Bindung, einer Amid-Bindung und einer Maleimid-Thiol-Bindung, die einen Spacer-Arm aufweisen, umfasst; das Ligieren oder das Synthetisieren die separate Ligierung des ACE2-Polypeptids an den Sense-Strang und den Antisense-Strang der shRNA umfasst.

9. Syntheseverfahren eines zielgerichteten Arzneimittels nCoVshRNA-2ACE2 nach Anspruch 1 oder 8, wobei das nCoVshRNA-2ACE2 den Sense-Strang und Antisense-Strang/shRNA, hergestellt mit SEQ ID NO: 7 bis SEQ ID NO: 58, oder ein Ligationsprodukt ACE2-shRNA mit dem ACE2, oder einen Liposomenkomplex der ACE2-shRNA umfasst; ACE2-shRNA 2ACE2-shRNA5 mit SEQ ID NO:49, 2ACE2-shRNA8 mit SEQ ID NO:30 umfasst; der Liposomenkomplex der ACE2-shRNA 2ACE2-shRNA5/Cholesterin, 2ACE2-shRNA8/Cholesterin, und/oder ACE2-shRNA-Liposom umfasst.

## Revendications

1. Procédé de synthèse *in vitro* d'un médicament à cible définie nCoVshRNA-2ACE2, comprenant les étapes suivantes consistant à : synthétiser un shRNA avec un brin sens et un brin antisens d'une séquence de RNAi, étendre une extrémité du brin sens et/ou du brin antisens constituant le shRNA, et ligaturer à un polypeptide ACE2 afin de synthétiser un médicament qui distribue le shRNA par le polypeptide ACE2, dans lequel la séquence de RNAi est un gène consensus conservé d'un virus COVID-19 et d'une souche variante de celui-ci, de sorte que le shRNA synthétisé avec le brin sens et le brin antisens de la séquence de RNAi est ciblé pour interférer avec le gène conservé, ce qui entraîne un effet de RNAi à large spectre.

2. Procédé de synthèse d'un médicament à cible définie nCoVshRNA-2ACE2 selon la revendication 1, dans lequel un processus de synthèse du shRNA comprend spécifiquement : la synthèse de deux brins sens et brins antisens complémentaires de 21 nt à 25 nt, et la synthèse d'une séquence de base qui agit comme un espaceur ; la ligature du brin sens et du brin antisens et de la séquence de base afin de former un petit shRNA en forme d'épingle à cheveux avec une boucle qui se trouve dans une position médiane et est séparée par la séquence de base ; et la ligature du polypeptide ACE2 à chaque brin simple du shRNA.

3. Procédé de synthèse d'un médicament à cible définie nCoVshRNA-2ACE2 selon la revendication 1 ou 2, pour la distribution ciblée du shRNA servant de cible de RNAi consensus d'un coronavirus ; comprenant en outre la ligature de l'ACE2 avec des propriétés antigéniques en un dimère ACE2 par le shRNA avec des propriétés d'adjuvant immunologique.

4. Procédé de synthèse d'un médicament à cible définie nCoVshRNA-2ACE2 selon la revendication 1 ou 3, dans lequel le brin sens et le brin antisens pour la synthèse du shRNA comprennent les séquences de RNAi SEQ ID NO: 7 à SEQ ID NO: 58 du virus COVID-19 ; et le brin sens et le brin antisens comprennent des séquences consensus préférées SEQ ID NO: 8 à SEQ ID NO: 10, SEQ ID NO: 16 à SEQ ID NO: 18, SEQ ID NO: 20 à SEQ ID NO: 22, SEQ ID NO: 30 à SEQ ID NO: 32, et SEQ ID NO: 41 à SEQ ID NO: 58 du virus COVID-19.

5. Procédé de synthèse d'un médicament à cible définie nCoVshRNA-2ACE2 selon la revendication 1 ou 4, dans lequel le brin sens et le brin antisens pour la synthèse du shRNA sont de préférence SEQ ID NO: 16 à SEQ ID NO: 18 et SEQ ID NO: 49 à SEQ ID NO: 51 ciblant un gène N du virus COVID-19, SEQ ID NO: 20 à SEQ ID NO: 22 et SEQ ID NO: 52 à SEQ ID NO: 54 ciblant un gène *ORFIab* du virus COVID-19, et SEQ ID NO: 30 à SEQ ID NO: 32 et SEQ ID NO: 56 à SEQ ID NO: 58 ciblant un gène S du virus COVID-19 ; le brin sens et le brin antisens pour la synthèse du shRNA sont plus préférablement SEQ ID NO: 16 et SEQ ID NO: 49 ciblant le gène N, SEQ ID NO: 21 et SEQ ID NO: 52 ciblant le gène *ORFIab,* et SEQ ID NO: 30 ciblant le gène S ; et le brin sens et le brin antisens pour la synthèse du shRNA sont encore plus préférablement SEQ ID NO: 49, et SEQ ID NO: 30.

6. Procédé de synthèse d'un médicament à cible définie nCoVshRNA-2ACE2 selon la revendication 1 ou 5, dans lequel un processus de synthèse du nCoVshRNA-2ACE2 comprend spécifiquement : la ligature du polypeptide ACE2 avec le shRNA et/ou le brin sens et le brin antisens afin de former un composé, ou l'insertion du shRNA et/ou du brin sens et du brin antisens dans l'ACE2 ou le polypeptide de celui-ci, et la ligature afin de former le composé ; et la modification du composé avec un liposome ou des nanoparticules lipidiques.

7. Procédé de synthèse d'un médicament à cible définie nCoVshRNA-2ACE2 selon la revendication 1 ou 6, dans lequel l'ACE2 comprend un ACE2 extracellulaire à des acides aminés 1 à 740, un ACE2 transmembranaire à des acides aminés 741 à 763, un ACE2 intracellulaire à des acides aminés 764 à 805, ou un polypeptide ACE2 à séquence d'acide aminé optimisée.

8. Procédé de synthèse d'un médicament à cible définie nCoVshRNA-2ACE2 selon la revendication 1 ou 6, dans lequel la ligature ou la synthèse comprend la ligature de l'ACE2 avec une extrémité 3' du brin antisens et une extrémité 5' ou une extrémité 3' du brin sens du shRNA ; la ligature ou la synthèse comprend la réalisation de la ligature par couplage chimique ou par couplage covalent avec une liaison carboxyhydrazone, une liaison disulfure, une liaison phosphodiester, une liaison phosphorodithioate, une liaison thioéther, une liaison oxime, une liaison amide et une liaison maléimide-thiol qui ont un bras espaceur ; la ligature ou la synthèse comprend la ligature du polypeptide ACE2 séparément sur le brin sens et le brin antisens du shRNA.

9. Procédé de synthèse d'un médicament à cible définie nCoVshRNA-2ACE2 selon la revendication 1 ou 8, dans lequel le nCoVshRNA-2ACE2 comprend le brin sens et le brin antisens/shRNA préparés avec SEQ ID NO: 7 à SEQ ID NO: 58, ou un produit de ligature ACE2-shRNA avec l'ACE2, ou un complexe de liposome de l'ACE2-shRNA ; l'ACE2-shRNA comprend 2ACE2-shRNAS ayant SEQ ID NO:49 - 2ACE2-shRNA8 ayant SEQ ID NO:30, le complexe de liposome de l'ACE2-shRNA comprend 2ACE2-shRNA5/cholestérol, 2ACE2-shRNA8/cholestérol, et/ou un liposome ACE2-shRNA.
